(19) Europäisches Patentamt
European Patent Office
Office européen des brevets

(11) EP 2 746 313 B1

(12) # EUROPÄISCHE PATENTSCHRIFT

(45) Veröffentlichungstag und Bekanntmachung des Hinweises auf die Patenterteilung:
**30.08.2017 Patentblatt 2017/35**

(51) Int Cl.:
***C08G 63/08*** *(2006.01)* ***C08G 63/90*** *(2006.01)*
***C08G 63/78*** *(2006.01)*

(21) Anmeldenummer: **12197885.2**

(22) Anmeldetag: **18.12.2012**

(54) **Vorrichtung und Verfahren zur Abtrennung eines cyclischen Diesters aus Polymerschmelzen**

Apparatus and method for separating a cyclic diester from polymer melts

Dispositif et procédé destinés à la séparation d'un diester cyclique depuis des masses fondues polymères

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**25.06.2014 Patentblatt 2014/26**

(73) Patentinhaber: **Uhde Inventa-Fischer GmbH**
**13509 Berlin (DE)**

(72) Erfinder:
- **Hagen, Rainer**
  **13465 Berlin (DE)**
- **Mühlbauer, Udo**
  **10119 Berlin (DE)**

(74) Vertreter: **Pfenning, Meinig & Partner mbB**
**Patent- und Rechtsanwälte**
**Theresienhöhe 11a**
**80339 München (DE)**

(56) Entgegenhaltungen:
**EP-A2- 2 055 730 WO-A1-2012/110117**
**US-A- 5 866 677**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 746 313 B1

## Beschreibung

Die vorliegende Erfindung betrifft eine Vorrichtung sowie ein Verfahren zur Abtrennung und Rückgewinnung eines cyclischen Diesters, insbesondere von Dilactid bzw. Glycolid aus Polymerschmelzen, die den cyclischen Diester als Verunreinigung beinhalten. Die Vorrichtung sowie das erfindungsgemäße Verfahren erlauben die Rückgewinnung des cyclischen Diesters mit hoher Ausbeute und gleichzeitig hoher Reinheit.

Bei der Polymerisation von PLA (Polyactid) enthält das Polymer aufgrund des chemischen Ring-Kettengleichgewichts stets Lactid in einer Konzentration, die abhängig von der Polymerisationstemperatur ist und zwischen etwa 1 und 5% beträgt. Dieser Wert ist unabhängig davon, ob das PLA durch Ringöffnungspolymerisation aus Lactid oder durch direkte Polykondensation aus Milchsäure hergestellt wird. Bei der Ringöffnungspolymerisation kann die Konzentration des Lactids auch höhere Werte annehmen, wenn die Reaktion bereits vor Erreichen des chemischen Gleichgewichts abgebrochen wird, z. B. durch Zugabe eines den Polymerisationskatalysator desaktivierenden Stoffs.

Lactidkonzentrationen im PLA von mehr als 0,5 Gew.-% machen das Polymer unbrauchbar für technische Zwecke. Sie führen bei der Verarbeitung von PLA in der Schmelze, wie Spinnen von Fäden, Gießen von Folien, Spritzgießen usw. zu Rauch, der zum Husten reizt, Vorrichtungen verschmutzt und korrodiert. Lactidhaltiges PLA-Granulat nimmt bei Lagerung an Umgebungsluft Feuchtigkeit auf, wobei Lactid zum linearen Dimeren der Milchsäure hydrolysiert wird. Bei der Verarbeitung aus der Schmelze führt dieses Hydrolyseprodukt aufgrund der dazu nötigen hohen Schmelzetemperatur von > 170°C (Schmelzpunkt von PLA) zu raschem Abbau der PLA-Ketten, so dass das Polymer technisch wichtige Eigenschaften wie Festigkeit, Transparenz etc. verliert und unbrauchbar wird.

Je niedriger die Restkonzentration des Lactids im PLA ist, desto haltbarer sind die daraus hergestellten Produkte und desto besser verhält es sich in der Verarbeitung. Deshalb strebt man eine möglichst niedrige Konzentration an Lactid in der entmonomerisierten PLA-Schmelze bei wirtschaftlich vertretbarem Aufwand an.

Aus diesen Gründen ist es erforderlich, aus dem PLA nach der Polymerisation das Lactid abzutrennen bis auf Restkonzentrationen unter 0,5 Gew.-%, vorzugsweise unter 0,2 Gew.-%. Im Stand der Technik geschieht das durch Verdampfen des nicht umgesetzten Monomers aus der Schmelze. Vakuum oder ein inertes Schleppgas erleichtern die Verdampfung, wobei häufig beide gleichzeitig verwendet werden.

Bekanntlich gelingt die Abtrennung von flüchtigen Komponenten aus einer Polymerschmelze umso vollständiger, je höher das Vakuum ist. Andererseits erschwert hohes Vakuum die Abscheidung des Lactids aus dem Dampfstrom. Im Stand der Technik wird deshalb das Vakuum bei der Entmonomerisierung meist so gewählt, dass eine Kondensation in flüssiger Form möglich ist. Dies begrenzt jedoch das anwendbare Vakuum auf Drücke die deutlich höher sind als der Druck am Tripelpunkt des Lactids. Damit ist die in der Schmelze erreichbare Lactidkonzentration ebenfalls begrenzt.

DE 196 30 121 A1 (Shimadzu) beschreibt die Monomerabtrennung aus PLA-Schmelze unter Vakuum mit Hilfe eines Dünnfilmverdampfers oder horizontaler Einzel- oder Doppelachsenreaktoren. Die Abscheidung des Monomers erfolgt durch Kondensation in flüssiger Form, bevor es in die Polymerisation zurückgeführt wird. Der verwendete Vakuumpumpentyp wird nicht beschrieben.

In EP 0 499 747 A2 (Novacor Chemicals) werden zur Monomerabtrennung Fallstrangentgaser, Entgasungsextruder oder Dünnschichtverdampfer vorgeschlagen. Die Dämpfe aus der Entgasung werden in einem oder mehreren hintereinandergeschalteten Kondensatoren niedergeschlagen. Zur Vakuumerzeugung werden ein-oder mehrstufige, nicht näher bezeichnete Aggregate verwendet, die ein Vakuum bis zu 0,002 atm. (= 2 mbar) erzeugen. Um den Partialdruck des abzutrennenden Lactids zu reduzieren und damit die Verdampfung zu erleichtern und den Restmonomergehalt im Polymer zu senken wird die Zugabe von Schleppmitteln wie Stickstoff, Toluol, Ethylbenzol als Möglichkeit genannt. Obwohl nicht explizit genannt, legt die Verwendung des Begriffs "Kondensator" nahe, daß die Dämpfe in flüssiger Form niedergeschlagen werden. Die Abscheidung in fester Form ist nicht erwähnt.

WO 98/36012 (Neste) bevorzugt zur Vakuumverdampfung einen Fallstrangentgaser, wobei die Polymerschmelze in Form von Fäden in einem offenbar nicht unter Vakuum stehenden Behälter nach unten fällt. Heißes Inertgas wie Stickstoff oder trockene Luft wird in den Entgaser eingeblasen, um die Verdampfung des Lactids von der Oberfläche der fallenden Fäden zu erleichtern. Das lactidhaltige heiße Gas wird nach Verlassen des Entgasungsapparats schnell auf 20-40°C abgekühlt, wobei Lactid als kristalliner Staub ausfällt. Vorzugsweise geschieht das in einer "Kristallisationskammer" durch Vermischen mit kalter Luft. Ohne Anwendung von Vakuum liegt der Druck bei der Lactidabscheidung weit über dem Tripelpunkt. Nachteil dieses Verfahrens ist die Vermischung des Lactids mit großen Mengen an Inertgas, die es erschweren, das Lactid vollständig zurückzugewinnen und die zusätzlichen Aufwand zur Trennung des Gases vom Lactidpulver erfordern (Zyklon, Gasfilter).

In EP 2 055 730 A2 (Hitachi) gehen die Dämpfe aus der PLA-Entmonomerisierung in einen Behälter zur Rückgewinnung, der mit einer Vakuumpumpe evakuiert wird. Dieser Behälter wird "mit bekannten Mitteln" gekühlt, so daß Lactid z. B. in Pulverform abgeschieden und als Rohstoff zur PLA-Herstellung wiederverwendet werden kann. Es werden keine Angaben zur technischen Ausführung des Behälters und zu Druck- und Temperaturbedingungen bei der Abscheidung gemacht. Ebenso fehlen Angaben zur technischen Ausführung der Vakuumpumpe. Die Entnahme des

Pulvers aus dem unter Vakuum stehenden Behälter ist nicht gelöst. Entnahme und Transport des Pulvers zur Rückführung in den Polymerisationsprozess ist schwierig auszuführen, wegen der großen spezifischen Oberfläche von Pulver bei der bekannt hohen Empfindlichkeit des Lactids gegen Luftfeuchte.

US 2009/0299018 A1 (Hitachi) beschreibt ein Verfahren und eine Vorrichtung zur Vakuumerzeugung bei der PLA-Entmonomerisierung. Der aus der Entmonomerisierung kommende Lactiddampf wird in einem Kondensator in direktem Kontakt mit einer Flüssigkeit niedergeschlagen, die als Hauptbestandteil Milchsäure enthält. Die Flüssigkeit fließt anschließend ab in einen atmosphärisch abgetauchten Sammelbehälter und wird von dort aus zum Kondensator zurückgeführt. Aus dem Sammelbehälter wird ein Teilstrom entnommen, um das niedergeschlagene Lactid dem Kreislauf zu entnehmen. Um eine Verstopfung des Kondensators durch polykondensierendes Lactid zu verhindern, wird wasserhaltige Milchsäure aus dem Kondensat der nachfolgenden Vakuumstufe dem Kreislauf zugeführt.

Das nötige Vakuum wird mit einer Sequenz aus 3 Strahlpumpen und einer nicht näher beschriebenen Vakuumpumpe erzeugt. Jede dieser Strahlpumpen ist ausgerüstet mit einem Einspritzkondensator, der mit einem atmosphärisch abgetauchten Kondensatsammelbehälter verbunden ist. Das hauptsächlich aus Wasser bestehende Kondensat wird im Kreislauf geführt, indem es in einen Dampferzeuger geht, der die jeweilige Strahlpumpe mit Treibdampf versorgt. Das Verfahren hat den Nachteil, daß das niedergeschlagene Lactid in einem Gemisch mit wasserhaltiger Milchsäure anfällt. Aus diesem Gemisch kann es wegen seiner Hydrolyseempfindlichkeit nicht in reiner Form isoliert werden und deshalb nicht unmittelbar als Rohstoff der Ringöffnungspolymerisation verwendet werden. Es reagiert mit Wasser und Milchsäure schnell zu linearen Oligomeren der Milchsäure, die wegen des zu hohen Endgruppenge-Endgruppengehalts unbrauchbar für die Ringöffnungspolymerisation sind.

Die Anwesenheit von wasserhaltiger Milchsäure bei der Kondensation des Lactids erlaubt es nicht, einen Druck unterhalb des Dampfdrucks des flüssigen Gemischs einzustellen. Damit ist das erreichbare Vakuum begrenzt und demzufolge die Restkonzentration an Lactid im entmonomerisierten PLA.

Nachteilig bei allen zuvor beschriebenen Verfahren ist jedoch, dass Lactid aus einer Entmonomerisierungseinheit für PLA nicht in derart hoher Reinheit zurückgewonnen werden kann, die es erlaubt, das Lactid erneut direkt in die Polymerisation zurück zu führen. Ebenso wenig ist aus dem Stand der Technik bekannt, Lactid in fester, kompakter Form, d. h. nicht als Pulver aus der Gasphase abzuscheiden und aus einem unter Vakuum stehenden Behälter zu entnehmen. Weiterhin ist aus dem Stand der Technik nicht bekannt, bei einer derartigen Entmonomerisierung eine Optimierung der Druckverhältnisse vorzunehmen, die gleichzeitig eine möglichst hohe Ausbeute, andererseits eine möglichst hohe Reinheit des zurückgewonnenen Dilactids ermöglicht.

Auch die US 5 866 677 A gibt ein Verfahren zur Rückgewinnung von Lactiddämpfen an, das einige Schwächen aufweist. Das Verfahren ist beispielsweise an die Polylactidherstellung gekoppelt. Es ist vorgesehen, dass der Lactiddampf direkt aus dem Reaktionsbehälter entnommen wird, in dem die Polymerisation von Lactid zu Polylactid durchgeführt wird. Dies wirkt sich nachteilig auf den Umsatz von Lactid zu Polylactid aus. Die zum Verfahren gehörige Vorrichtung weist die gleichen Nachteile auf.

Die WO 2012/110117 A1 offenbart ein Verfahren zur Herstellung von Polymilchsäure, bei dem die rohe PLA-Schmelze, die durch Ringöffnungspolymerisation (ROP) entsteht, gereinigt und mit Hilfe eines einstufig durch eine Pumpe erzeugten Vakuums von noch enthaltenem monomerem Lactid befreit wird. Das gasförmige Lactid wird dann durch mehrfaches Kristallisieren und wieder Aufschmelzen oder durch Desublimation erst von Verunreinigungen befreit, bevor es dem Polymerisationsprozess wieder zugeführt wird.

Die EP 2 055 730 A2 ist auf eine Vorrichtung und ein Verfahren zur Entgasung einer Polylactidschmelze gerichtet. Es wird beschrieben, dass dazu ein Druck verwendet werden sollte, der kleiner als der Druck während der Polymerisationsreaktion ist. Die Weiterbehandlung der aus der Schmelze entfernten Lactiddämpfe wird nicht beschrieben.

Es ist somit Aufgabe der Erfindung das abgetrennte Lactid, aus Gründen der Wirtschaftlichkeit, in möglichst reiner Form und mit größtmöglicher Ausbeute zurückzugewinnen und für die Polymerisation wiederzuverwenden. Wenn das Lactid in reiner Form zurückgewonnen wird, soll es im Falle der Ringöffnungspolymerisation direkt dem Rohstoff der Polymerisation zugesetzt werden. Im Falle der direkten Polykondensation der Milchsäure soll dieses Lactid nach Zugabe von Wasser durch Hydrolyse in Milchsäure zurückverwandelt und direkt dem Rohstoff für die Polykondensation zugesetzt werden.

Ein Problem, das dabei zu lösen ist, bilden flüchtige Abbauprodukte des PLAs, die in der Polymerisation entstehen. Sie verdampfen in der Vakuumentmonomerisierung zusammen mit dem Lactid und sollen möglichst nicht mit diesem zusammen kondensieren. Dabei kann es sich um Milchsäure und deren lineare Oligomere handeln, um die Hauptkomponenten des thermischen PLA-Abbaus Kohlenstoffmonoxid, -dioxid, Wasser und Acetaldehyd, sowie um Spuren von höheren Aldehyden wie Propionaldehyd, Butyraldehyd, Crotonaldehyd und Isovaleraldehyd, weiter um Pentadien, diverse Furane wie 2,5-Dimethylfuran, Carbonsäuren, besonders Essigsäure und Propionsäure in niedrigen Konzentrationen. Weitere Nebenprodukte können aus Katalysatoren, Stabilisatoren und anderen Additiven entstehen, die vor oder nach Abschluss der Polymerisation zugesetzt werden. Manche dieser Begleitstoffe würden das abgeschiedene Lactid verunreinigen, indem sie es verfärben, geruchlich beeinträchtigen oder unter Ringöffnung chemisch verändern. Das Lactid kann dann erst nach einer Reinigung wie Umkristallisation oder Rektifikation als Rohstoff in die Polymerisation zurückgeführt werden. Im Falle der Hydrolyse des Lactids kann die entstandene Milchsäure erst nach einer Reinigung in die Polykondensation zurückgeführt werden. Dies verursacht erhöhten technischen und wirtschaftlichen Aufwand. Es ist deshalb eine weitere Aufgabe der vorliegenden Erfindung, solche Verunreinigung des abgeschiedenen Lactids durch Nebenprodukte zu vermeiden.

Ein weiteres dabei zu lösendes Problem bildet die Vakuumerzeugung für die Entmonomerisierung. In der vorliegenden Erfindung wird hohes Vakuum benötigt, um besonders niedrige Lactidkonzentrationen im entmonomerisierten PLA zu erreichen und das verdampfte Lactid in fester Form abzuscheiden. Dem Fachmann ist bekannt, dass bei der Kondensation oder Desublimation aufgrund des Dampfdrucks umso größere Restmengen an flüchtigen Komponenten wie Lactid im Gasstrom verbleiben, je höher das Vakuum gewählt wird. Außerdem haben die Erfinder die Erfahrung gemacht, dass es außerordentlich schwer ist, Lactiddämpfe zu kondensieren oder zu desublimieren, ohne daß geringe Restmengen als festes oder flüssiges Aerosol zusammen mit der unvermeidlichen Leckluft in die Vakuumpumpen gelangen und sich dort ablagern. Auch wenn diese Restmengen sehr gering sind, genügen sie wegen der Korrosivität und Abrasivität des Lactids, um übliche mechanische Vakuumpumpen (z. B. Roots-, Drehschieber-, Schraubenpumpen) in kurzer Zeit zu zerstören. Deshalb bestand eine weitere Aufgabe darin, Vakuumaggregate zu finden, die gegen Lactid in fester oder flüssiger Form unempfindlich sind und nicht teure Schutzmaßnahmen erfordern, wie die Herstellung aus korrosions- und abrasionsfesten Werkstoffen.

Eine Nebenforderung an geeignete Vakuumpumpen ist die Skalierbarkeit für große Anlagenkapazitäten, insbesondere das Vermeiden von mehrsträngiger, paralleler Ausführung. Diese würde die Anzahl der Vakuumpumpen proportional zur Anlagenkapazität ansteigen lassen und so die erwünschte Kostendegression bei großen Anlagenkapazitäten verhindern.

Diese Aufgaben werden mit einer Vorrichtung mit den Merkmalen des Patentanspruchs 1 sowie mit einem Verfahren mit den Merkmalen des Patentanspruchs 15 gelöst. Die jeweiligen weiteren Patentansprüche stellen dabei vorteilhafte Weiterbildungen dar.

Im Rahmen der Beschreibung der vorliegenden Erfindung wird auf die nachfolgenden Definitionen verwiesen, die im Rahmen der zur Beschreibung der Erfindung verwendeten Terminologie durchgängig einheitlich zu verstehen sind:

> Desublimieren: Direkter Übergang eines Stoffes vom dampfförmigen Zustand in den festen Zustand bei Drücken und Temperaturen unterhalb des Tripelpunkts, d. h. ohne dazwischen den flüssigen Zustand zu durchlaufen. Die Umkehrung von Sublimieren.

Abscheidevorrichtung oder Lactidabscheider: Im Folgenden ist darunter ein verfahrenstechnischer Apparat zu verstehen, in dem sich dampfförmiger Diester, z.B. Lactid an gekühlten Oberflächen in fester Form abscheidet und von denen es durch Abschmelzen bei Drücken und Temperaturen oberhalb des Tripelpunkts zurückgewonnen werden kann.

Tripelpunkt: Punkt im Druck-Temperatur-Diagramm eines Reinstoffes, in dem alle drei Phasen, feste, flüssige und dampfförmige, koexistieren. Im Tripelpunkt treffen sich die Phasengrenzlinien fest/flüssig, flüssig/dampfförmig und fest/dampfförmig. Für reines L-Lactid liegt dieser Punkt bei 96,9°C und 1,4 mbar. Im Rahmen dieser Erfindung ist dieser Wert nur als Beispiel zu sehen, er hängt ab von der Zusammensetzung des abgeschiedenen Lactids in dem vorgestellten Verfahren. Sowohl der Gehalt des Lactids an den Stereoisomeren L-Lactid, meso-Lactid und D-Lactid wirkt sich auf den Tripelpunkt aus, als auch Nebenprodukte der PLA-Polymerisation, die in der Entmonomerisierung zusammen mit dem Lactid verdampfen oder sublimieren. Hier sind Milchsäure und andere zyklische oder lineare Oligomere des PLA zu nennen sowie Abbauprodukte der PLA-Polymerisation, die in der Beschreibung bereits genannt wurden. Verunreinigungen in einem Reinstoff wie L-Lactid senken generell dessen Schmelzpunkt. Wegen der weitgehenden Druckunabhängigkeit des Schmelzpunkts gilt das auch für den Tripelpunkt. Verunreinigungen im Lactid verschieben demnach den Tripelpunkt in Fig. 2 nach links, auf einer gedachten Verlängerung der Dampfdruckkurve a, zu niedrigeren Temperaturen und Drücken im Vergleich zum reinen L-Lactid.

Entgasen, Entgaser: Abtrennung bzw. Apparat zur Abtrennung eines flüchtigen Stoffs aus einer Schmelze durch Verdampfen oder Verdunsten, d. h. unterhalb des Siedepunkts der Schmelze und oberhalb oder unterhalb des Siedepunkts des reinen flüchtigen Stoffs. Entgasen und Entgaser wird hier synonym mit Verdampfen und Verdampfer verwendet.

Fallstrangentgaser: Kontinuierlicher Entgaser, in dem der Polymerschmelzestrom durch eine Vielzahl von Düsenbohrungen in Stränge (oder auch Fäden) aufgeteilt wird, die im senkrechten Fall den Innenraum eines evakuierten Behälters durchlaufen. In der Fallzeit zwischen Austritt der Schmelze aus der Bohrung und Auftreffen auf dem Behälterboden verdampft enthaltenes Monomer. Die Schmelze wird vom Behälterboden kontinuierlich abgezogen und ausgetragen.

Atmosphärische Abtauchung: Dem Fachmann bekannte Vorrichtung, um Flüssigkeit kontinuierlich aus einem unter Vakuum stehenden System ohne Pumpe auf Atmosphärendruck zu bringen und auszuschleusen. Im Falle von Wasser als Flüssigkeit ist der Vakuum führende Behälter in mehr als 10 m Höhe über einem sog. Abtauchbehälter (Fig.

1. Nr. 4 und 6) angeordnet, der Wasser enthält und unter Atmosphärendruck steht. Vom Vakuum führenden Behälter führt eine Rohrleitung nach unten in den Abtauchbehälter, wo sie unterhalb des Wasserspiegels endet. Infolge des Vakuums wird Wasser in diesem Rohr so weit nach oben gesaugt, bis die hydrostatische Höhe der Wassersäule der Druckdifferenz zwischen den beiden Behältern die Waage hält. Im Falle von Wasser beträgt diese Höhe maximal 10 m über dem Spiegel im Abtauchbehälter. Wasser kann so ungehindert aus dem Vakuum auf Atmosphärendruck abfließen. Flüssigkeiten mit höherer Dichte als Wasser, wie z. B. Milchsäure, erreichen den Zweck bereits mit geringerer hydrostatischer Höhe.

Erfindungsgemäß wird somit eine Vorrichtung zur Abtrennung und Rückgewinnung eines cyclischen Diesters der allgemeinen Formel I

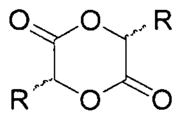

Formel I

wobei R ausgewählt ist aus Wasserstoff oder linearen oder verzweigten aliphatischen Resten mit 1 bis 6 Kohlenstoffatomen, aus Polymerschmelzen, enthaltend den Diester der allgemeinen Formel I, beschrieben, die

a) mindestens eine Entmonomerisierungsvorrichtung zur Entfernung des Diesters der allgemeinen Formel I in gasförmigem Aggregatzustand aus der Polymerschmelze,

b) mindestens eine, der mindestens einen Entmonomerisierungsvorrichtung nachgeschaltete und mit der mindestens einen Entmonomerisierungsvorrichtung in fluidischer Verbindung stehende Abscheidevorrichtung zur Abscheidung des Diesters der allgemeinen Formel I, in der der Diester der allgemeinen Formel I über den festen Aggregatzustand in den flüssigen Aggregatzustand überführt wird, wobei die mindestens eine Abscheidevorrichtung derart ausgelegt ist, dass eine Abscheidung des Diesters der allgemeinen Formel I durch Abkühlen auf Temperaturen unterhalb der Tripelpunkttemperatur

i) bei Drücken oberhalb des Tripelpunktdrucks des Diesters der Formel I an einer auf unterhalb der Tripelpunkttemperatur temperierten Oberfläche der mindestens einen Abscheidevorrichtung erfolgt, wobei der Diester der Formel I in den festen Aggregatzustand überführt wird, wobei der Druck in der mindestens einen Entmonomerisierungsvorrichtung auf 1,4 bis 100 mbar sowie der Druck in der mindestens einen Abscheidevorrichtung während der Überführung in den festen Aggregatzustand auf 1,4 bis 100 mbar eingestellt wird, oder

ii) bei Drücken unterhalb des Tripelpunktdrucks des Diesters der Formel I an einer auf unterhalb der Tripelpunkttemperatur temperierten Oberfläche der mindestens einen Abscheidevorrichtung erfolgt, wobei der Diester der Formel I desublimiert und somit in den festen Aggregatzustand überführt wird, wobei der Druck in der mindestens einen Entmonomerisierungsvorrichtung auf höchstens 1,4 mbar, bevorzugt von 0,01 bis 1,4 mbar, besonders bevorzugt von 0,1 bis 1,4 mbar sowie der Druck in der mindestens einen Abscheidevorrichtung während der Desublimation auf höchstens 1,4 mbar, bevorzugt von 0,01 bis 1,4 mbar, besonders bevorzugt von 0,1 bis 1,4 mbar eingestellt wird,

sowie

c) mindestens eine, der mindestens einen Abscheidevorrichtung nachgeschaltete und mit der mindestens einen Abscheidevorrichtung in fluidischer Verbindung stehende Vorrichtung zur Erzeugung eines Vakuums,
wobei die mindestens eine Abscheidevorrichtung über mindestens einen bodenseitigen Auslass verfügt, dem mindestens ein Sammeltank für den Diester der allgemeinen Formel I nachgeschaltet ist und
wobei der mindestens einen Vorrichtung zur Erzeugung eines Vakuums mindestens eine Vorrichtung zur Erzeugung eines Vorvakuums vorgeschaltet ist, die mit der mindestens einen Vorrichtung zur Erzeugung eines Vakuums in fluidischer Verbindung steht,

umfasst.

Die Vorrichtung gemäß der vorliegenden Erfindung beinhaltet damit zumindest drei wesentliche Bestandteile, nämlich eine Entmonomerisierungsvorrichtung, eine Abscheidevorrichtung sowie eine Vorrichtung zur Erzeugung eines Vakuums. Die Vorrichtungen sind dabei in einer Reihe nacheinander geschaltet, sodass mittels des von der Vorrichtung

zur Erzeugung des Vakuums erzeugten Unterdrucks Gase oder Dämpfe aus der Entmonomerisierungsvorrichtung über die Abscheidevorrichtung geleitet werden können. In der Abscheidevorrichtung erfolgt an geeigneten gekühlten Oberflächen eine Desublimation bzw. eine Abscheidung des Diesters der allgemeinen Formel I in festem Aggregatzustand. Damit ist die Abscheidetemperatur nach unten nicht begrenzt durch den Schmelzpunkt des Lactids. Der Austrag des so abgeschiedenen Diesters aus der Abscheidevorrichtung erfolgt jedoch im flüssigen Aggregatzustand.

Die erfindungsgemäße Vorrichtung ermöglicht eine effiziente Abtrennung des cyclischen Diesters, d. h. es kann ein Großteil des gasförmig abgetrennten cyclischen Diesters in der Abscheidevorrichtung durch die Desublimation oder Abscheidung im festem Aggregatzustand rückgewonnen werden. Zudem ist es mit der erfindungsgemäßen Vorrichtung ebenso möglich, den cyclischen Diester in hoher Reinheit rückzugewinnen, sodass sich der cyclische Diester beispielsweise unmittelbar zur weiteren Verwendung, insbesondere für eine Ringöffnungspolymerisation eignet.

Eine bevorzugte Ausführungsform sieht vor, dass die mindestens eine Abscheidevorrichtung über Mittel mit kühlbaren und/oder erwärmbaren Oberflächen, insbesondere Rohre, Rohrbündel, Platten, Plattenregister und/oder Wände, etc. an denen die Desublimation oder Abscheidung des Diesters der allgemeinen Formel I erfolgt, verfügt.

Die mind. eine Abscheidevorrichtung, insbesondere die Mittel mit kühlbaren oder erwärmbaren Oberflächen der Abscheidevorrichtung können durch aktive Versorgung mit entsprechenden kühlen bzw. warmen Medien auf die jeweiligen Betriebstemperaturen gebracht werden. Hierbei ist es vorteilhaft, wenn der mindestens einen Abscheidevorrichtung mindestens ein Dreiwegeventil zur Versorgung insbesondere der Mittel mit kühlbaren oder erwärmbaren Oberflächen, mit einem kühlenden oder erwärmenden Medium vorgeschaltet und/oder mindestens ein Dreiwegeventil zur Abführung eines kühlenden oder erwärmenden Mediums nachgeschaltet ist. Gemäß dieser Ausführungsform kann die jeweilige Abscheidevorrichtung somit wahlweise mit einem kühlenden oder erwärmenden Medium versorgt werden, sodass ein entsprechender Wechselbetrieb der Abscheidevorrichtung möglich ist.

Insbesondere ist es bevorzugt, dass die erfindungsgemäße Vorrichtung mind. zwei Abscheidevorrichtungen umfasst, die alternierend betrieben werden können und über ein Dreiwegeventil mit der Entmonomerisierungsvorrichtung und über ein Dreiwegeventil mit mindestens einer Vorrichtung zur Erzeugung eines Vakuums in fluidischer Verbindung stehen. Diese Ausführungsform sieht beispielsweise vor, dass bei mind. zwei Abscheidevorrichtungen die eine Abscheidevorrichtung mit einem kühlenden Medium versorgt wird und somit der Abscheidung von cyclischem Diester zur Verfügung steht. Die andere Abscheidevorrichtung kann dabei regeneriert werden, indem beispielsweise ein erwärmendes Medium dieser Abscheidevorrichtung zugeführt wird und der darin abgeschiedene cyclische Diester aufgetaut bzw. geschmolzen und somit in den flüssigen Aggregatzustand überführt wird. Die Mehrzahl der Abscheidevorrichtungen, insbesondere zwei Abscheidevorrichtungen, können dabei über eine gemeinsame fluidische Verbindung jeweils mit der Entmonomerisierungsvorrichtung und jeweils mit der Vorrichtung zur Erzeugung eines Vakuums verbunden sein, sodass beispielsweise die Vorrichtung, in der gerade eine Abscheidung des cyclischen Diesters durchzuführen ist über die beiden Weichen sowohl mit der Entmonomerisierungsvorrichtung, als auch mit der Vorrichtung zur Erzeugung des Vakuums verbindbar ist.

Über den bodenseitigen Auslass kann bei der Regenerierung der Abscheidevorrichtung, d. h. bei Erwärmen und somit Abschmelzen des zuvor in festem Aggregatzustand abgeschiedenen cyclischen Diesters der in der Abscheidevorrichtung angesammelte Diester bodenseitig ausgetragen und einem Sammeltank zugeführt werden.

Der Sammeltank kann beispielsweise der zeitweiligen Lagerung des Dilactids dienen, ebenso ist die Möglichkeit gegeben, dass der Sammeltank direkt mit einer Polymerisationseinrichtung verbunden ist und der gesammelte cyclische Diester über den Sammeltank dieser Polymerisationsvorrichtung zugeführt werden kann.

Vorzugsweise ist die mindestens eine Vorrichtung zur Erzeugung eines Vakuums eine Strahlvakuumpumpe, insbesondere eine Dampfstrahlvakuumpumpe oder eine Kaskade aus mindestens zwei, bevorzugt mindestens drei Strahlvakuumpumpen, insbesondere Dampfstrahlvakuumpumpen. Insbesondere die Anordnung mehrerer Strahlvakuumpumpen zu einer Kaskade ermöglicht die Erzeugung von niedrigen Drücken, was eine effiziente Abtrennung des cyclischen Diesters aus der Polymerschmelze ermöglicht. Besonders vorteilhaft hierbei ist, dass die Kaskade aus mindestens 2 Strahlvakuumpumpen ohne Zwischenkondensation ausgeführt ist. Bezüglich einer derartigen Kaskade mit Zwischenkondensation wird auf die US 2009/0299018 verwiesen, die insbesondere in Figur 2 sowie zugehöriger Beschreibung eine entsprechende Kaskade angibt. Eine derartige Kaskade ohne Zwischenkondensation wird auch in der in Figur 1 dargestellten erfindungsgemäßen Vorrichtung eingesetzt. Diese Kaskade ist dort mit den Bezugszeichen 3a, 3b, 3c gekennzeichnet. Die Zwischenkondensation stellt einen zusätzlichen Aufwand dar, der in der Regel notwendig ist, um die Leistung der Strahlpumpen nicht zu stark einzuschränken oder den Treibmittelverbrauch zu begrenzen. Es wurde gefunden, dass im vorliegendem Fall bis zu 3 Strahlpumpen ohne Zwischenkondensation betrieben werden können, wobei Leistung und Treibmittelverbrauch akzeptabel sind.

Bevorzugte Entmonomerisierungsvorrichtungen sind dabei ausgewählt aus der Gruppe bestehend aus Fallfilmverdampfern, Fallstrangverdampfern, Entgasungsextrudern, Vakuumknetern, Dampfabscheidern und/oder Dünnschichtverdampfern.

Weitere Vorteile ergeben sich, wenn der mindestens einen Vorrichtung zur Erzeugung eines Vakuums mindestens ein Kondensator, insbesondere ein Oberflächenkondensator nachgeschaltet ist, der mit der mit einem Auslass

der mindestens einen Vorrichtung zur Erzeugung eines Vakuums in fluidischer Verbindung steht. Diese Ausführungsform sieht vor, dass die aus der mind. einen Vorrichtung zur Erzeugung eines Vakuums austretenden Prozessdämpfe bzw. Brüden auskondensiert werden, wobei sich kondensierbare Bestandteile, wie beispielsweise ggf. enthaltene cyclische Diester gemäß der allgemeinen Formel I oder die entsprechenden, den cyclischen Diester der Formel I bildenden Säuren, auskondensieren und somit entfernen lassen.

Es ist zudem möglich, dass dem mindestens einen Kondensator mindestens ein Kondensatsammelbehälter nachgeschaltet ist, der mit dem mindestens einen Kondensator in fluidischer Verbindung steht, insbesondere mit Rohrleitung verbunden ist.

Eine weitere Ausführungsform sieht vor, dass dem mindestens einen Kondensator mindestens eine Vorrichtung zur Erzeugung eines Vorvakuums, insbesondere mindestens eine Wasserringpumpe vorgeschaltet ist, die mit dem mindestens einen Kondensator in fluidischer Verbindung steht. Das Vorschalten einer weiteren Vorrichtung zur Erzeugung eines Vorvakuums vor die eigentlichen Vorrichtungen zur Erzeugung des Vakuums verbessert die Qualität und Stabilität des Vakuums. Mit der Vorrichtung zur Erzeugung des Vorvakuums, insbesondere einer Wasserringpumpe, können typischerweise Drücke von ca. 40 mbar erzeugt werden. Mit den eigentlichen Vorrichtungen zur Erzeugung des Vakuums, beispielsweise der Kaskade von Strahlpumpen, lassen sich typischerweise Drücke von 1 mbar und weniger erzeugen.

Eine weiter bevorzugte Ausführungsform sieht vor, dass die erfindungsgemäße Vorrichtung eine Reinigungskolonne, die insbesondere eine Strippkolonne ist, umfasst. Diese Reinigungskolonne kann dem mindestens einen Kondensatsammelbehälter, der mindestens einen Vorrichtung zur Erzeugung eines Vakuums und/oder dem mindestens einen Kondensator nachgeschaltet sein und steht mit den jeweiligen zuvor genannten Bestandteilen in fluidischer Kommunikation. Die Reinigungskolonne weist dabei kopfseitigen Einlass für Kondensat, einen bodenseitigen Gaseinlass, einen kopfseitigen Gasauslass sowie einen bodenseitigen Flüssigkeitsauslass auf. Mittels dieser Reinigungskolonne können aus dem Kondensat darin lösliche flüchtige Bestandteile, insbesondere flüchtige Abbauprodukte des cyclischen Diesters der allgemeinen Formel 1, wie beispielsweise flüchtige Aldehyde, insbesondere Acetaldehyd etc. abgetrennt werden und über den Gasauslass abgezogen werden.

Eine weitere Ausführungsform der erfindungsgemäßen Vorrichtung sieht vor, dass der mindestens einen Reinigungskolonne, insbesondere der Strippkolonne, der mindestens einen Vorrichtung zur Erzeugung eines Vakuums, dem mindestens einen Kondensator und/oder dem mindestens einen Kondensatsammelbehälter mindestens ein Dampferzeuger nachgeschaltet ist, der einen Flüssigkeitszulauf aufweist, der mit der mindestens einen Reinigungskolonne, insbesondere der Strippkolonne über den Flüssigkeitsauslass, einem Auslass der mindestens einen Vorrichtung zur Erzeugung eines Vakuums, dem mindestens einen Kondensator und/oder dem mindestens einen Kondensatsammelbehälter in fluidischer Verbindung steht. Dem Dampferzeuger kann somit das erzeugte Kondensat zugeführt und erneut verdampft werden. Diese Ausführungsform ist insbesondere dann bevorzugt, wenn die Vorrichtung zur Erzeugung eines Vakuums eine Dampfstrahlvakuumpumpe ist. Insofern kann das Wasser, das in dampfförmiger Form zum Betreiben der Dampfstrahlvakuumpumpe verwendet wird, in Kreislauf. Damit wird der Bedarf an Frischwasser zum Betrieb der Strahlpumpen entscheidend reduziert, desgleichen das anfallende Abwasser, was die Wirtschaftlichkeit des Strahlpumpenbetriebs erhöht.

Weiter ist es bevorzugt, wenn der mindestens eine Dampferzeuger

a) mindestens einen Brenner umfasst, der mindestens eine Zuführung für Brennstoff sowie eine Zuführung von gasförmigem Oxidationsmittel für den Brennstoff, die mit dem kopfseitigen Gasauslass der Reinigungskolonne, insbesondere der Strippkolonne in fluidischer Verbindung steht, aufweist; und/oder

b) eine Abführung für erzeugten Dampf aufweist, die mit einer Dampfstrahlvakuumpumpe oder einer Kaskade aus mindestens zwei, bevorzugt mindestens drei Dampfstrahlvakuumpumpen verbunden ist, so dass die Dampfstrahlvakuumpumpe oder die Kaskade aus mindestens zwei, bevorzugt mindestens drei Dampfstrahlvakuumpumpen durch mit vom Dampferzeuger erzeugten Dampf betrieben werden kann.

Mit einem derartigen Dampferzeuger können beispielsweise die flüchtigen, aus der Polyesterschmelze abgetrennten Bestandteile direkt dem Brenner zugeführt und dort oxidiert bzw. verbrannt und damit entsorgt werden. Die dabei entstehende zusätzliche Abwärme kann zur Erzeugung von Dampf genutzt werden. Für den Fall, dass es sich bei den Vorrichtungen zur Erzeugung des Vakuums um Dampfstrahlvakuumpumpen handelt, kann der vom Dampferzeuger erzeugte Dampf direkt zum Betrieb dieser Vorrichtungen verwendet werden.

Die Erfindung betrifft ebenso ein Verfahren zur Abtrennung und Rückgewinnung eines cyclischen Diesters der allgemeinen Formel I

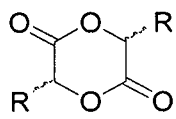

Formel I

wobei R ausgewählt ist aus Wasserstoff oder linearen oder verzweigten aliphatischen Resten mit 1 bis 6 Kohlenstoffatomen, aus Polymerschmelzen, enthaltend den Diester der allgemeinen Formel I, mit einer erfindungsgemäßen Vorrichtung, bei dem die Polymerschmelze in mindestens einer Entmonomerisierungsvorrichtung mit Vakuum beaufschlagt und der Diester der allgemeinen Formel I durch Übergang in den gasförmigen Aggregatzustand aus der Polymerschmelze zumindest teilweise oder vollständig abgetrennt und der entfernte gasförmige Diester der allgemeinen Formel I in mindestens einer Abscheidevorrichtung durch Abkühlen auf Temperaturen unterhalb der Tripelpunkttemperatur

a) bei Drücken oberhalb des Tripelpunktdrucks des Diesters der Formel I an einer auf unterhalb der Tripelpunkttemperatur temperierten Oberfläche der mindestens einen Abscheidevorrichtung in den festen Aggregatzustand überführt wird, wobei der Druck in der mindestens einen Entmonomerisierungsvorrichtung auf 1,4 bis 100 mbar sowie der Druck in der mindestens einen Abscheidevorrichtung während der Überführung in den festen Aggregatzustand auf 1,4 bis 100 mbar eingestellt wird oder

b) bei Drücken unterhalb des Tripelpunktdrucks des Diesters der Formel I an einer auf unterhalb der Tripelpunkttemperatur temperierten Oberfläche der mindestens einen Abscheidevorrichtung desublimiert und somit in den festen Aggregatzustand überführt wird, wobei der Druck in der mindestens einen Entmonomerisierungsvorrichtung auf höchstens 1,4 mbar, bevorzugt von 0,01 bis 1,4 mbar, besonders bevorzugt von 0,1 bis 1,4 mbar sowie der Druck in der mindestens einen Abscheidevorrichtung während der Desublimation auf höchstens 1,4 mbar, bevorzugt von 0,01 bis 1,4 mbar, besonders bevorzugt von 0,1 bis 1,4 mbar eingestellt wird

und wobei dass anschließend eine Verflüssigung und ein Austrag des Diesters der allgemeinen Formel I aus der mindestens einen Abscheidevorrichtung erfolgt, wobei Diester der allgemeinen Formel I aus der mindestens einen Abscheidevorrichtung über mindestens einen bodenseitigen Auslass ausgetragen wird und in einem nachgeschalteten Sammeltank gesammelt wird, wobei dem Vakuum, das durch mindestens eine Vorrichtung zur Erzeugung eines Vakuums erzeugt wird, ein Vorvakuum vorgeschaltet ist, das durch mindestens eine Vorrichtung zur Erzeugung eines Vorvakuums, die mit der mindestens einen Vorrichtung zur Erzeugung eines Vakuums in fluidischer Verbindung steht, erzeugt wird.

Die Erfindung betrifft somit zwei alternative Ausführungsformen. Einerseits kann die Abscheidung des Diesters der Formel I unterhalb des Drucks am Tripelpunkt des Diesters (und gleichzeitig unterhalb der Tripelpunkttemperatur des Diesters der Formel I) erfolgen, in diesem Falle erfolgt eine Desublimation des Diesters der Formel I auf den kalten Teilen der Abscheidevorrichtung. Der Diester der Formel I schlägt sich mit einer dem Rauhreif ähnlichen Beschaffenheit auf den gekühlten Oberflächen der Abscheidevorrichtung nieder.

Bevorzugt erfolgt die Abscheidung jedoch oberhalb des Drucks am Tripelpunkt des Diesters der Formel I, hierbei liegt jedoch die Temperatur unterhalb der Tripelpunkttemperatur der Formel I. In diesem Falle erfolgt zunächst eine Kondensation zu feinen flüssigen Tröpfchen (Nebel), die sich anschließend auf den kalten Teilen der Abscheidevorrichtung zu einer kompakten Schicht verfestigen.

Der Austrag des abgeschiedenen Diesters aus der mindestens einen Abscheidevorrichtung erfolgt nach Aufschmelzen des Diesters.

Das erfindungsgemäße Verfahren ermöglicht überraschenderweise die Abscheidung des Diesters der Formel I in hoher Ausbeute bei gleichzeitig hoher Reinheit. Zudem bewerkstelligt das vorliegende Verfahren eine einfache Anreicherung des Diester, der in fester Form in der Abscheidevorrichtung erhalten wird. Nach Erhöhen des Drucks im Behälter über den Tripelpunkt des abgeschiedenen Diesters kann dieser durch Abschmelzen auf einfache Art und Weise in flüssiger Form der Abscheidevorrichtung entnommen werden.

Eine bevorzugte Ausführungsform des Verfahrens sieht vor, dass der Diester der allgemeinen Formel I nach Desublimation oder Abscheidung im festen Aggregatzustand in den flüssigen Aggregatzustand überführt und bodenseitig in der mindestens einen Abscheidevorrichtung gesammelt, und anschließend aus der mindestens einen Abscheidevorrichtung ausgetragen wird. Der erhaltene Diester weist eine hohe Reinheit auf und kann beispielsweise direkt einer Polykondensationsreaktion zur Herstellung eines Polymeren aus dem Diester, beispielsweise durch ringelöffnende Polymerisation überführt werden. Ggf. kann der Diester nach Austrag auch in einen Sammeltank überführt und dort zwischenzeitlich gelagert werden.

Die Mittel der mindestens einen Abscheidevorrichtung zur Desublimation oder Überführung in den festen Aggregatzustand des Diesters der allgemeinen Formel I werden vorzugsweise alternierend mit einem auf unterhalb der

Tripelpunkttemperatur temperierten Medium und zur Überführung in den flüssigen Aggregatzustand mit einem auf oberhalb der Tripelpunkttemperatur tur temperierten Medium durchströmt. Gemäß dieser Ausführungsform ist ein Wechselbetrieb der Abscheidevorrichtung möglich, sodass während eines Zyklus, während dem die Abscheidevorrichtung mit einem kalten Medium durchströmt wird, eine Abscheidung des Diesters bei den jeweiligen Bedingungen möglich ist. Bei Durchlaufen des Zyklus, bei dem die Abscheidevorrichtung erwärmt wird, kann der Diester aufgetaut werden und sammelt sich am Boden der Abscheidevorrichtung, von wo aus ein Austrag über den Auslass möglich ist.

Zudem ist es bevorzugt, wenn innerhalb der mindestens einen Abscheidevorrichtung während der Desublimation oder Überführung in den festen Aggregatzustand des Diesters der allgemeinen Formel I ein im Vergleich zur Überführung in den flüssigen Aggregatzustand verminderter Druck eingestellt wird, wobei bevorzugt der Druck bei der Überführung in den flüssigen Aggregatzustand auf mindestens 2,5 mbar, weiter bevorzugt von 2,5 bis 1050 mbar, besonders bevorzugt von 10 bis 50 mbar eingestellt wird.

Das Aufschmelzen des abgeschiedenen Diesters kann auch beim gleichen Druck wie beim Abscheiden erfolgen. In diesem Fall beträgt der Druck mindestens 2,4 mbar, bevorzugt 2,5 - 50, besonders bevorzugt zwischen 2,5 und 10 mbar.

Weiter ist es bevorzugt, wenn mindestens zwei Abscheidevorrichtungen umfasst sind, die alternierend betrieben werden. Diese Verfahrensführung erlaubt eine quasi kontinuierliche Abtrennung von Diester aus der Polymerschmelze.

Für den Fall, dass eine Desublimation des Diesters der Formel I durchgeführt wird, d. h. bei Drücken unterhalb des Tripelpunktdrucks gearbeitet wird, ist es nötig, dass der Druck in der Entmonomerisierung naturgemäß mindestens gleich ist, wegen des Druckverlustes in Strömungsrichtung, bevorzugt 0,01 mbar bis 1 mbar über dem Druck im Abscheider beträgt. Insbesondere wird der Druck in der mindestens einen Entmonomerisierungseinrichtung auf höchstens 1,4 mbar, bevorzugt von 0,01 bis 1,4 mbar, besonders bevorzugt von 0,1 bis 1,4 mbar sowie der Druck in der mindestens einen Abscheidevorrichtung während der Desublimation auf höchstens 1,4 mbar, bevorzugt von 0,01 bis 1,4 mbar, besonders bevorzugt von 0,1 bis 1,4 mbar eingestellt.

Andererseits ist ebenso eine Überführung des Diesters in den festen Aggregatzustand oberhalb des Tripelpunktes des Diesters der Formel I möglich. Hierbei liegt der Druck in der mindestens einen Entmonomerisierungsvorrichtung bei höchstens 1050 mbar, bevorzugt von 1,4 bis 1050 mbar, besonders bevorzugt von 1,4 bis 100 mbar sowie der Druck in der mindestens einen Abscheidevorrichtung während der Überführung in den festen Aggregatzustand auf höchstens 1050 mbar, bevorzugt von 1,4 bis 1050 mbar, besonders bevorzugt von 1,4 bis 100 mbar.

Die zuvor genannten Ausführungsformen, die absolute Drücke benennen, sind dabei insbesondere für Dilactid als Diester der allgemeinen Formel I vorteilhaft, d.h. R = Methyl.

Absolut betrachtet funktioniert das erfindungsgemäße Verfahren daher in einem Arbeitsbereich von 0,01 mbar bis 1050 mbar, bevorzugt 0,1 mbar bis 100 mbar, mit der Maßgabe, dass während des Abscheidevorgangs die Kühlelemente in der Abscheidevorrichtung auf Temperaturen gekühlt werden, bei denen eine Verfestigung des Lactids erfolgen kann.

Die Dämpfe aus den Strahlpumpen werden bevorzugt in einen Kondensator geleitet. Der Wasserdampf, der zum Betrieb der Strahlpumpen verwendet wird, kondensiert zusammen mit den Restmengen des zyklischen Diesters und Abbauprodukten aus der Entmonomerisierung. Übrig bleibt Restgas, das nicht kondensierbar ist, z.B. Leckluft aus dem Prozess. Das Restgas wird von der Vorvakuumpumpe abgesaugt und an die Umgebung oder in die Kesselfeuerung befördert.

Weiter bevorzugt wird das Kondensat kopfseitig in die mindestens eine Reinigungskolonne, bevorzugt die mindestens eine Strippkolonne, aufgegeben und im Gegenstrom mit einem Reinigungsgas, bevorzugt Luft beaufschlagt. Diese Ausführungsform ermöglicht die Abtrennung gelöster Abbauprodukte des PLAs aus dem Kondensat, wie beispielsweise Aldehyde, insbesondere Acetaldehyd, die somit entsprechend abgetrennt und entsorgt werden können.

Weiter ist vorteilhaft, wenn das aus der mindestens einen Reinigungskolonne, bevorzugt der mindestens einen Strippkolonne bodenseitig entnommene Kondensat dem mindestens einen Verdampfer zugeführt und dort verdampft wird, wobei der entstehende Dampf zum Betreiben der Dampfstrahlvakuumpumpe oder der Kaskade aus mehreren Dampfstrahlvakuumpumpen verwendet wird.

Das aus dem kopfseitigen Gasauslass der Reinigungskolonne entnommenen Reinigungsgas kann zusammen mit dem Oxidationsmittel, insbesondere Luft oder Sauerstoff, für den Brennstoff in den mindestens einen Brenner des mindestens einen Dampferzeugers aufgegeben werden.

Insbesondere eignet sich das Verfahren zur Abtrennung von Lactid bzw. Glycolid aus Polyesterschmelzen, insbesondere Polylactid oder Polyglycolid und/oder Copolyestern hiervon. Im Falle von Lactiden bzw. Polylactiden kann jedes beliebige Stereoisomer der Lactide aus einer entsprechenden Polyesterschmelze abgetrennt werden.

Die vorliegende Erfindung wird anhand der beigefügten Figur sowie der nachfolgenden Ausführungen näher erläutert, ohne die Erfindung auf die dort dargestellten speziellen Parameter zu beschränken.

Figur 1 zeigt einen beispielhaften Aufbau einer erfindungsgemäßen Vorrichtung zur Durchführung eines erfindungsgemäßen Verfahrens.

Figur 2 zeigt ein nicht stoffspezifisches Phasendiagramm eines erfindungsgemäß eingesetzten Diesters.

Die erfindungsgemäße Vorrichtung umfasst dabei eine Entmonomerisierungsvorrichtung 1, die über einen Schmelzeeinlass C sowie einen Schmelzeauslass C1 für einen Polyester, beispielsweise Polylactid, verfügt. Die Entmonomerisierungsvorrichtung 1 weist einen Gasauslass C2 auf, der mit einem Dreiwegeventil 10 verbunden ist. Über das Dreiwegeventil 10 kann wahlweise eine Abscheidevorrichtung 2a oder 2b mit einem Gasstrom aus der Entmonomerisierungsvorrichtung 1 versorgt werden. Eine jede der Abscheidevorrichtungen 2a und 2b ist dabei identisch aufgebaut und weist im Inneren gekühlte Oberflächen auf. Diese Oberflächen sind über eine Weiche 11a bzw. 11b mit einem kalten Medium A bzw. einem warmen Medium B versorgbar. In Figur 1 ist ein Wechselbetrieb der beiden Abscheidevorrichtungen 2a und 2b dargestellt, hierbei wird momentan die Abscheidevorrichtung 2a über die Weiche 11a mit einem kalten Medium A versorgt, während die Abscheidevorrichtung 2b über die Weiche 11b mit einem warmen Medium B versorgt wird. Nach Durchlaufen des Kühlkörpers wird das Medium A bzw. B über eine Weiche 12a bzw. 12b aus der jeweiligen Abscheide-vorrichtung 2a bzw. 2b ausgetragen. Im Falle der Figur 1 erfolgt momentan eine Aufgabe des aus der Entmonomerisierungsvorrichtung 1 stammenden Gasstroms C2 in die Abscheidevorrichtung 2a. Hierbei erfolgt eine Abscheidung des im Gasstrom enthaltenen cyclischen Diesters, beispielsweise des Lactids auf den gekühlten Oberflächen der Abscheidevorrichtung 2a in festem Zustand. Beide Abscheidevorrichtungen 2a und 2b sind dabei über eine Weiche 13 an eine Kaskade von Dampfstrahlvakuumpumpen 3a, 3b und 3c angeschlossen. Wie aus Figur 1 ersichtlich, wird momentan lediglich die Abscheidevorrichtung 2a mit Vakuum über die Weiche 13 beaufschlagt. Die Abscheidevorrichtung 2b befindet sich in Figur 1 dargestellten Zustand momentan in Regenerierung, indem die gekühlten Oberflächen der Abscheidevorrichtung 2b mit einem warmen Medium B durchströmt werden. Bereits auf den nunmehr beheizten Oberflächen befindlicher fester cyclischer Diester, beispielsweise Lactid, wird somit erwärmt und bei entsprechend hohen Drücken und hohen Temperaturen in die flüssige Phase überführt. Der flüssige Diester tropft somit an den beheizten Oberflächen herab und sammelt sich am Boden der Abscheidevorrichtung 2b und kann dort über eine Stellweiche 14 ausgetragen und einem Sammelbehälter 9 zugeführt werden. Der Sammelbehälter 9 verfügt über einen bodenseitigen Auslass I.

Die Kaskade der Dampfstrahlvakuumpumpen, 3a, 3b, 3c wird jeweils mit Dampf D versorgt. Der Dampfstrahlvakuumkaskade ist ein Kondensator 4 nachgeschaltet, in dem aus den Dampfstrahlvakuumpumpen ausgetragener Dampf sowie nicht in den Abscheidevorrichtungen 2a bzw. 2b abgeschiedene Bestandteile kondensiert werden können. Der Kondensator 4 wird dabei mit einem kühlenden Medium E betrieben und enthält im Inneren angeordnete gekühlte Oberflächen, z.B. ein Rohrbündel. Dem Kondensator 4 nachgeschaltet ist eine Wasserringpumpe 5 zur Erzeugung eines Vorvakuums, das beispielsweise 40 mbar betragen kann. Entsprechende Abgase H können ohne weitere Reinigung an die Umwelt abgegeben werden oder in die Feuerung des Dampferzeugers D verbrannt werden.

Zudem beschreibt Figur 1 eine Ausführungsform, bei der dem Kondensator ein Sammeltank 6 nachgeschaltet ist, in dem entsprechende wässrige Kondensate gesammelt und bevorratet werden können. Der Sammeltank 6 ist über eine Pumpe 15 mit einer Strippkolonne 7 verbunden, die eine kopfseitige Zuführung 18 für das Kondensat aufweist. Die Zuführung kann beispielsweise einen Rieselkopf oder Sprühdüse aufweisen, sodass das Kondensat gleichmäßig über den Kolonnenquerschnitt verteilt wird. Zudem verfügt die Strippkolonne einen in Bodennähe angeordneten Gaseinlass 19, mit dem über ein Gebläse 16 ein Gas F, beispielsweise Luft, in die Strippkolonne 7 eingeblasen werden kann. Somit wird Luft im Gegenstrom zum oben eingerieselten Kondensat geführt, wodurch das Kondensat von flüchtigen Bestandteilen befreit werden kann. Diese können über den kopfseitigen Gasauslass 20 aus der Strippkolonne 7 abgeführt werden, während sich das gereinigte Kondensat am Boden sammelt und dort bodenseitig 21 aus der Strippkolonne 7 ausgetragen werden kann. Über eine Pumpe 17 ist eine Zuführung des Kondensates zu einem Dampferzeuger 8 möglich, in dem aus dem Kondensat Dampf D erzeugt wird, der erneut zur Betreibung der Kaskade von Dampfstrahlvakuumpumpen 3a, 3b, 3c verwendet werden kann. Die aus der Strippkolonne 7 kopfseitig 20 abgeführten Gase können in die Zufuhr für Oxidationsmittel 23 eines Gasbrenners 22 zugespeist werden, der mit einem Brennstoff G betrieben wird. Somit können flüchtige Bestandteile, die aus dem Kondensat in der Strippkolonne 7 abgetrennt wurden, verbrannt werden. Die Abluft H des Dampferzeugers 8 wird an die Umwelt abgegeben.

Im Folgenden wird eine beispielhafte bevorzugte Ausführungsform zur Ausführung eines erfindungsgemäßen Verfahrens angegeben, das die Erfindung anhand des Beispiels der Abtrennung von Lactid aus einer Lactidenthaltenden Polylactid(PLA)-Schmelze darstellt.

Eine monomerhaltige PLA-Schmelze wird nach der Polymerisation in bekannter Weise durch Verdampfen im Vakuum von Lactid befreit. Dies kann kontinuierlich oder diskontinuierlich geschehen. Beispiele sind statische Methoden wie das freie Abfließen der PLA-Schmelze im Vakuum über Oberflächen von Einbauten wie geneigte Bleche, statische Mischer oder Stoffaustauschpackungen oder frei fallende Schmelzefilme, -stränge oder -fäden in Vakuumkammern, mechanisch erzwungene Bewegung der Schmelze wie in einem Entgasungsextruder, einem Vakuumkneter oder Verteilung in dünner Schicht über beheizte Oberflächen mit Hilfe von mechanischen Wischern (Dünnschichtverdampfer). All diesen Methoden ist gemeinsam, dass sie die Verdampfung durch Vakuum und/oder Schleppgas, große Oberflächen, Wärmezufuhr und Mischen begünstigen und so einen lactidhaltigen Dampfstrom erzeugen.

Im erfindungsgemäßen Verfahren wird das zunächst dampfförmige Lactid nach Herausführen aus der Vorrichtung zur Entmonomerisierung durch Abkühlen im Vakuum in fester Form abgeschieden. Entmonomerisierung und Abscheidung des Lactids erfolgen etwa bei demselben Druck, wobei ein geringes Druckgefälle von der Entmonomerisierung zur Abscheidung durch Anordnung des Vakuumsystems nach der Abscheidung aufrecht erhalten wird, um einen Transport durch Strömung in dieser Richtung zu bewirken. Die Abkühlung der Dämpfe erfolgt an gekühlten Oberflächen. Die gekühlten Oberflächen sind in einem unter Vakuum stehenden Behälter angeordnet, der hier als Lactidabscheider bezeichnet wird.

Das Zumischen von kaltem Inertgas zum Lactiddampfstrom zu Kühlungszwecken wird vermieden, weil dabei der nicht kondensierbare Gasstrom zu hoch wird, der letztlich vom Vakuum auf Umgebungsdruck zu verdichten ist und dabei viel Energie verbraucht. Ohne Zusatz von Inertgas ist das Lactid leichter aus der Gasphase abzuscheiden und in kompakter Form (nicht als Staub) zu erhalten.

Die Abscheidung kann bei Drücken unterhalb des Drucks am Tripelpunkt des Lactids geschehen, d. h. also durch Desublimation. Die Abscheidung des Lactids durch Desublimation ergibt ein besonders reines Lactid, da es direkt in kristalliner Form anfällt, die Verunreinigungen weitgehend ausschließt. Dieses Lactid kann wegen seiner Reinheit direkt in den Polymerisationsprozess oder nach Hydrolyse in die Polykondensation zurückgeführt werden. Die oben genannten Begleitstoffe, die zu Verfärbung oder Ringöffnung des Lactids führen würden, können auf besonders niedrigem Konzentrationsniveau gehalten werden. Die im Vergleich zur Kondensation niedrigere Abscheidetemperatur bei der Desublimation verringert die Gefahr der Ringöffnung des Lactids durch letzte Reste dieser Begleitstoffe wie Milchsäure oder deren linearem Dimer. Nachteilig ist die Abscheidung auf den gekühlten Oberflächen als lose, rauhreifartige Schicht mit teilweise reduzierter Haftung.

Das Verfahren funktioniert aber auch bei Drücken oberhalb des Drucks am Tripelpunkt, wobei die Abscheidetemperatur unterhalb des Lactidschmelzpunkts liegen muss. Diese Abscheidetemperatur ist unterhalb der Temperatur am Tripelpunkt des reinen L-Lactids zu suchen und einzustellen und hängt ab von den genannten Verunreinigungen im Lactid. Diese verschieben den Schmelzpunkt zu niedrigeren Werten hin. Bei Abkühlung des Dampfes an den gekühlten Oberflächen kondensiert das Lactid zunächst in flüssiger Form als Tröpfchen (Nebel), die nach Auftreffen auf die gekühlten Flächen des Lactidabscheiders feste Schichten bilden.

Diese Art der Abscheidung hat den Vorteil, dass die Tröpfchen gut an den gekühlten Oberflächen haften und kompakte Schichten bilden. Abtragen von der festen Oberfläche und Mitreißen von Lactid durch die Strömung der nicht abgeschiedenen Gase und Dämpfe kommt hier nicht vor, im Gegensatz zu den raureifartigen Schichten, die man bei der Abscheidung unterhalb des Drucks am Tripelpunkt erhält. Infolgedessen gelangt weniger mitgerissener Lactidstaub in die nachfolgende Vakuumanlage, die Lactidverluste sind geringer und ebenso die korrosive und abrasive Belastung der Vakuumpumpen.

Sowohl bei der Abscheidung oberhalb des Drucks am Tripelpunkt als auch unterhalb davon ist sicherzustellen, dass die Temperatur an den gekühlten Oberflächen unterhalb der Schmelztemperatur des Lactids liegt.

Unabhängig davon, ob der Abscheidedruck oberhalb oder unterhalb des Drucks am Tripelpunkts liegt, bietet die Wahl der Abscheidetemperatur ein gewisses Optimierungspotential: Je niedriger diese Temperatur gewählt wird, z. B. durch Verwendung von Kaltwasser oder flüssigem Kältemittel, desto vollständiger ist die Abscheidung des Lactids aus der Gasphase. Gleichzeitig steigt jedoch auch die Konzentration von Nebenprodukten im abgeschiedenen Lactid, weil deren Kondensations- oder Desublimationstemperatur unterschritten wird. Es gilt deshalb eine Abscheidetemperatur zu finden, die eine hohe Reinheit des Lactids ermöglicht bei tragbaren Lactidverlusten im Abgas des Lactidabscheiders.

Die Entnahme des abgeschiedenen Lactids aus dem Abscheider erfolgt bevorzugt durch periodisches Erhöhen des Drucks im Behälter und der Temperatur an den mit Lactid belegten Rohren und Platten über die Temperatur am Tripelpunkt des Lactids. Das führt zum Abschmelzen des festen Lactids von den belegten Oberflächen. Dabei sammelt sich das flüssige Lactid im Behälterboden. Mit Hilfe von an sich bekannten Flüssigkeitspumpen wird es aus dem Vakuum auf Umgebungsdruck gefördert und zwischengelagert. Die technische Ausführung wird im Beispiel unten genauer beschrieben.

Im Falle, dass der Druck während des Abscheidens bereits oberhalb des Drucks am Tripelpunkt gelegen hat, ist eine Druckerhöhung zum Abschmelzen im Prinzip nicht nötig. Es empfiehlt sich jedoch, sowohl in diesem Falle als auch bei der Lactidabscheidung unterhalb des Drucks am Tripelpunkt, den Druck beim Abschmelzen soweit zu erhöhen, dass das Temperaturfenster zwischen Schmelzpunkt und Siedepunkt des Lactids ausreichend breit ist, um unbeabsichtigtes Wiederverdampfen infolge von technisch unvermeidlichen Schwankungen der Temperaturführung im Behälter zu verhindern.

Bevorzugt werden Strahlpumpen für die Vakuumerzeugung verwendet. Sie enthalten keine beweglichen Teile und sind deshalb besonders robust gegen Lactidablagerungen in fester oder flüssiger Form. Sie können ohne Schwierigkeiten aus korrosionsfestem Material gefertigt werden. Sie haben sich auch als resistent gegen Abrasion durch Lactid-Aerosole erwiesen. Dies konnte der Fachmann nicht erwarten, da die Strömungsgeschwindigkeit des Dampfes in diesen Pumpen bekanntlich Überschallgeschwindigkeit erreicht und feste Partikel bei hohen Geschwindigkeiten besonders abrasiv sind. Strahlpumpen eignen sich deshalb in besonderer Weise, in Kombination mit den beschriebenen Lactidabscheidern Lactid aus PLA-Schmelze zu entfernen. Durch Hintereinanderschalten von mehreren Strahlpumpen kann man auf einfache Weise auch Drücke unterhalb des Tripelpunkts und so besonders niedrige Restkonzentrationen im PLA erreichen.

Die Vakuumerzeugung mit Dampfstrahlpumpen erfordert als Treibmittel Wasserdampf. Um den Verbrauch der Anlage an Frischwasser und die Abgabe von Abwasser an die Umwelt niedrig zu halten, ist es vorteilhaft, das Wasser nach den Strahlpumpen möglichst vollständig zurückzugewinnen und wieder für die Dampferzeugung zu nutzen. Es wurde gefunden, dass eine solche Kreislaufführung des Wassers auf Probleme stößt. Die Dampfstrahlpumpen konnten dabei nach kurzer Zeit das vorgesehene Vakuum nicht mehr stabil halten oder gar nicht erreichen. Um den Einsatz von Strahlpumpen möglich zu machen, mussten die Ursache des Problems und geeignete Maßnahmen zur Abhilfe gefunden werden.

Überraschend wurde jedoch gefunden, dass trotz einer atmosphärenseitig vor den Strahlpumpensatz geschalteten Wasserringpumpe, die für das erforderliche Vorvakuum sorgt und zusammen mit dem Restgas auch flüchtige Nebenprodukte aus dem Wasserkreislauf entfernt, offenbar noch gelöste Nebenprodukte im Wasser verbleiben und in den Dampferzeuger gelangen. Dadurch verschlechtert sich die Dampfqualität bei geschlossenem Wasserkreislauf schnell und führt zu den genannten Vakuumproblemen. Diese werden gelöst durch das vorteilhafte Anordnen einer Strippkolonne vor dem Dampferzeuger. Diese Kolonne entzieht dem Wasser flüchtige, jedoch in Wasser lösliche und damit anreicherungsfähige Nebenprodukte der PLA-Polymerisation mit Hilfe eines Luftstroms. Wasser und Luft werden in der Strippkolonne im Gegenstrom geführt, so dass die mit Nebenprodukten beladene Abluft am oberen Ende der Kolonne entweicht. Am unteren Ende fällt das gereinigte Wasser an und wird dem Dampferzeuger zugeführt. Dieser Prozess stellt keine Ansprüche an die Qualität der verwendeten Luft, so dass staubfrei gefilterte Umgebungsluft verwendet werden kann.

Die Abluft der Kolonne kann anschließend der Verbrennungsluft für eine in Polymerisationsanlagen sowieso benötigte Kesselfeuerung zugeführt werden. Auf diese Weise werden die Nebenprodukte auf unschädliche und kostengünstige Weise beseitigt. Dies ist unter Umweltgesichtspunkten vorteilhaft, da einige der Abbauprodukte sehr geruchsintensiv sind.

Fig. 2 zeigt ein Phasendiagramm eines reinen Stoffes, der hier der Diester ist. P und T sind Druck und Temperatur des Diesters in einem geschlossenen Gefäß. Die eingezeichneten Kurven (a, b, c) trennen 3 Gebiete voneinander ab - die feste, flüssige und gasförmige Phase (Dampf). Die Kurven treffen sich im sogenannten Tripelpunkt, an dem alle 3 Phasen miteinander im Gleichgewicht stehen. Der Tripelpunkt ist charakteristisch für den betrachteten Reinstoff.

‚a' ist die Dampfdruckkurve, auf ihr steht siedende Flüssigkeit mit Dampf im Gleichgewicht. 'b' ist die Sublimationsdruckkurve, auf der festes Sublimat im Gleichgewicht mit Dampf steht. ‚c' ist die Schmelzdruckkurve, sie zeigt die Schmelztemperatur des Diesters in Abhängigkeit vom Druck. Wie bei den meisten Stoffen ist diese Abhängigkeit nur schwach ausgebildet und bei den Drücken, bei denen erfindungsgemäß gearbeitet wird, zu vernachlässigen.

Eine Druck- und Temperaturskala fehlt auf den Achsen der Fig. 2, da das erfindungsgemäße Vorgehen hier nur prinzipiell erläutert werden soll. Insbesondere hängen der Wert des Tripelpunkts und der Verlauf der Gleichgewichtskurven von der Art des Diesters ab. Im Falle des Lactids hat auch die Zusammensetzung aus D, L und meso-Lactid einen Einfluss auf die genaue Lage.

Punkt W kennzeichnet Druck und Temperatur des Diester-Dampfes, der aus der Entmonomerisierung 1 der Fig. 1 kommt, im Eingang des Abscheiders 2a.

Die gekühlten Oberflächen im Abscheider haben eine Temperatur, die durch Punkt X gekennzeichnet ist. Punkt X liegt bezüglich Druck und Temperatur unterhalb des Tripelpunktes des Diesters. Infolge der Abkühlung an den Oberflächen sinkt die Dampftemperatur und erreicht die Sublimationsdruckkurve 'b'. Dort scheidet sich fester Diester auf den gekühlten Oberflächen ab, der nach einer gewissen Zeit, die von der Schichtdicke abhängt, die Temperatur der Oberfläche annimmt (Punkt X). Dieser Vorgang setzt sich fort, bis die verfügbaren Oberflächen im Abscheider 2a mit so dicken Schichten bedeckt sind, dass der Wärmeübergang vom Dampf an die Oberflächen stark abnimmt. Infolge dessen steigt die Dampftemperatur am Auslass an, die Dampfzufuhr in Abscheider 2a wird beendet und der Dampf aus der Entmonomerisierung in Abscheider 2b geleitet.

Zur Regenerierung von Abscheider 2a wird der Druck im Behälter erhöht, z.B. durch Einleiten von Inertgas, bis Punkt Y erreicht ist. In Punkt Y befindet sich der abgeschiedene Diester bei einer Temperatur unterhalb der TripelpunktTemperatur, und bei einem Druck oberhalb des Tripelpunkt-Drucks. Nun wird die Zufuhr des Kühlmediums A unterbrochen und auf das Heizmedium B umgeschaltet. Der feste Diester, der an den Oberflächen haftet, erwärmt sich, schmilzt, fließt von den Oberflächen ab und sammelt sich in flüssiger Form am Boden des Abscheiders. Beim Abfließen von den jetzt beheizten Oberflächen nimmt der Diester die Temperatur dieser Oberflächen an, die durch Punkt Z gekennzeichnet ist.

Nachdem der geschmolzene Diester in Behälter 9 abgelassen wurde, wird der Abscheider 2a wieder auf den im Behälter 2b herrschenden Druck bei W evakuiert und auf die Temperatur am Punkt X gekühlt. Er steht danach wieder zur Beladung zur Verfügung, sobald Abscheider 2b voll ist.

Der Weg von Punkt W zu Punkt X illustriert die Abscheidung des Diesters unterhalb des Drucks am Tripelpunkt.

Die Vorrichtung und das Verfahren gemäß Erfindung gestatten auch eine Abscheidung des Diesters oberhalb des Drucks am Tripelpunkt. Diese Arbeitsweise zeigt der Weg von Punkt W' zu X' in Fig. 2.

Punkt W' kennzeichnet Druck und Temperatur des aus der Entmonomerisierung kommenden Diester-Dampfes im Eingang des Abscheider 2a. Die gekühlten Oberflächen im Abscheider haben eine Temperatur, die durch Punkt X' gekennzeichnet ist. Punkt X' liegt bezüglich des Drucks oberhalb des Tripelpunkts, bezüglich der Temperatur jedoch darunter.

Infolge von Abkühlung an den gekühlten Oberflächen im Abscheider sinkt die Dampftemperatur bei konstantem Druck und erreicht zunächst den Bereich der flüssigen Phase. Der Diester kondensiert in Form von feinen Tröpfchen aus, die sich an den kalten Oberflachen niederschlagen und dort durch weiteres Absinken ihrer Temperatur einfrieren. Es bildet sich eine feste Schicht des Diesters auf den gekühlten Oberflächen, die in einer gewissen Zeit, die von der Schichtdicke abhängt, die Temperatur dieser Oberflächen annimmt (Punkt X').

Die Dicke dieser Schicht wächst stetig an, solange Dampf aus der Entmonomerisierung 1 dem Abscheider 2a zugeführt wird. Wenn der Wärmeübergang durch die Schichtdicke zu stark behindert wird, steigt die Temperatur am Dampfauslass an. Die Dampfzufuhr aus der Entmonomerisierung wird unterbrochen und der Dampf wird in den zweiten Abscheider 2b geleitet.

Zur Regenerierung wird der Druck im Abscheider 2a bei konstanter Temperatur erhöht, z.B. durch Einleiten von Inertgas, bis Punkt Y erreicht ist. Das weitere Vorgehen entspricht der Regenerierung wie sie bei der Abscheidung des Diesters unterhalb des Drucks am Tripelpunkt beschrieben wurde.

Im Prinzip ist die Regenerierung nach der Abscheidung des Diesters oberhalb des Drucks am Tripelpunkt auch ohne Druckerhöhung möglich. Dabei wird der abgeschiedene Diester, dessen Temperatur durch X' gekennzeichnet ist, bei konstantem Druck durch Temperaturerhöhung auf einen Wert, der durch Z' gekennzeichnet ist, abgeschmolzen. Nach Abzug des flüssigen Diesters in Behälter 9 steht der Abscheider für einen neuen Zyklus zur Verfügung.

Wie aus Fig. 2 zu entnehmen ist, ist die Temperaturspanne, in der der Diester flüssig vorliegt, deutlich kleiner als bei erhöhtem Druck. Dadurch steigt die Gefahr, bei ungenauer Temperatur- oder Druckregelung den Diester wieder zu verdampfen oder einzufrieren und so den Zweck des Verfahrens zu verfehlen.

Oberhalb des Drucks am Tripelpunkt kann alternativ zu dem erfindungsgemäßen Verfahrens auch ein Kondensator eingesetzt werden, der kontinuierlich und somit einfacher zu betreiben ist. Unterhalb des Drucks am Tripelpunkt gibt es diese Alternative nicht und das erfindungsgemäße Verfahren ist das einzig mögliche. Für einen Kondensator gilt jedoch die oben genannte Einschränkung, dass in der Nähe oberhalb des Tripelpunkts die Temperaturspanne zwischen fester Phase und Dampfphase sehr klein ist, so dass bei unzureichender Regelung der Temperatur oder des Drucks entweder Einfrieren des Kondensators oder Ausbleiben der Kondensation eintritt. In der Nähe oberhalb des Tripelpunkts ist deshalb kein sicherer (störungsfreier) Betrieb eines Kondensators möglich und das erfindungsgemäße Verfahren ist trotz des diskontinuierlichen Betriebs vorteilhafter.

**Beispiel 1:**

Dieses Beispiel illustriert das Verfahren, wobei die Lactidabscheidung unterhalb des Drucks am Tripelpunkt geschieht.

In einer Anlage zur PLA-Herstellung durch Ringöffnungspolymerisation ist das Abgassystem der Entmonomerisierung gemäß Fig. 1 ausgeführt. Der Abgasstutzen des Apparats zur Entmonomerisierung 1 ist verbunden mit zwei parallel geschalteten Lactidabscheidern 2a, 2b, von denen jeweils einer in Betrieb ist und der andere sich in Regeneration befindet. Der Ausgang des in Betrieb befindlichen Lactidabscheiders 2a ist verbunden mit einem Satz aus 3 hintereinander geschalteten Dampfstrahlvakuumpumpen 3a, 3b, 3c. Das Vorvakuum für diesen Satz erzeugt eine Wasserringpumpe 5, welche den Restgasstrom auf Umgebungsdruck verdichtet.

Die Lactidabscheider 2a, 2b sind vakuumfeste Behälter, die gekühlte Rohre und Platten enthalten. Rohre und Platten füllen den unter Vakuum stehenden Innenraum so aus, dass die hindurchstreichenden am Eintritt noch 190°C heißen Dämpfe und Gase in intensiven Kontakt mit den gekühlten Oberflächen kommen, ohne dass Kurzschlussströmungen entstehen. Auf der Außenseite der von innen mit Wasser auf 40°C gekühlten Rohre und Platten schlägt sich Dilactid in fester Form nieder und bildet Schichten, deren Dicke im Laufe des Betriebs beständig wächst. Die wachsende Schicht reduziert den Wärmeübergang vom Gas an die gekühlten Oberflächen, so dass die Abscheideleistung des Apparats im Verlauf eines Zyklus' geringer wird. Bevor nennenswerte Mengen an nicht abgeschiedenem Lactid im Gasauslass des Lactidabscheiders erscheinen, erkennbar an einem Temperaturanstieg an dieser Stelle, wird der lactidhaltige Gasstrom aus dem Entmonomerisierer auf den zweiten Lactidabscheider umgeschaltet, der inzwischen regeneriert wurde und bereitsteht. Im ersten Lactidabscheider wird das Vakuum durch Einleiten von Stickstoff bis auf 20 mbar aufgefüllt, so dass der Druck oberhalb des Tripelpunkts liegt. Die Zufuhr von Kühlwasser wird durch die von heißem Druckwasser B mit 120°C ersetzt. Dabei schmelzen die Lactidschichten von den Oberflächen ab, flüssiges Lactid sammelt sich am Boden des Lactidabscheiders und wird in einen beheizten Vorratstank abgelassen. Von dort wird es wieder der Polymerisation zugeführt. Gegen Ende des Abschmelzvorgangs wird der Apparat wieder unter volles Vakuum gesetzt

und steht für einen weiteren Desublimationszyklus zur Verfügung.

Das aus dem Lactidabscheider kommende Restgas, das aus Leckluft und den flüchtigen Abbauprodukten des PLAs besteht, wird abgesaugt durch 3 in Serie geschaltete Strahlpumpen 3a - 3c, die mit Wasserdampf von 3 bar abs. als Treibmittel arbeiten. Der Pumpensatz wird so betrieben, dass am Gasauslass des Lactidabscheiders 2a ein Druck von 0,5 mbar abs. anliegt. Am Gasauslass des Entmonomerisierers 1 beträgt der Druck infolge von Verlusten im Lactidabscheider 1,5 mbar. Der Dampf aus dem Strahlpumpensatz wird ohne Zwischenkondensation nach der 3. Stufe einem Oberflächenkondensator 4 zugeführt, der mit Kaltwasser auf 24°C gehalten wird. Der Druck beträgt hier 30 mbar. Eine Wasserringpumpe 5 verdichtet das nach der Kondensation verbliebene Restgas auf Umgebungsdruck und fördert es an die Atmosphäre.

Das auf dem Druckniveau von 30 mbar kondensierte Wasser fließt aufgrund einer Höhendifferenz von > 10 m in einen unter Atmosphärendruck stehenden Sammelbehälter 6 ab. Von dort wird es zum Kopf einer Strippkolonne 7 gepumpt, wo es im Gegenstrom zu aus der Umgebung angesaugter Luft über eine Füllkörperpackung fließt. Am unteren Ende der Kolonne ist das Wasser so weit gereinigt, dass es dem Dampfkessel 8 zugeführt werden kann, der den Treibdampf erzeugt. Die Abluft aus der Strippkolonne 7 wird in die Zuluft der Kesselfeuerung eingeleitet und verbrannt.

Das in den Lactidabscheidern anfallende Lactid hat nach Aufschmelzen und Ablassen aus dem Sammelbehälter 9 eine Carboxylgruppen-Konzentration von 20 mmol/kg und bildet eine klare, farblose Schmelze. Es wird ohne weitere Reinigung dem frischen Lactid zugesetzt, das als Rohstoff für die Ringöffnungspolymerisation in der Anlage dient. Die Rückführung verändert nicht die Farbe des amorphen PLA-Granulats und die mittlere Molmasse, gemessen über die intrinsische Viskosität gegenüber dem Betrieb der Anlage ohne diese Rückführung.

Die nach der Ringöffnungspolymerisation in die Entmonomerisierung eintretende PLA-Schmelze hat eine Lactid-Konzentration von 4,5 %. Diese Konzentration hat in dem aus der Entmonomerisierung 1 kommenden PLA C1 auf eine Lactidkonzentration von 0,15 % abgenommen, gemessen mit Gaschromatographie, nach Abschrecken der Schmelze in Wasser und Granulieren. An einer Wasserprobe aus Behälter 6 wird der Säuregehalt durch Titration (analytische Methode 1) gemessen und in Lactid umgerechnet. Diese Lactidkonzentration ist ein Maß für den Lactidverlust durch Mitreißen aus den Abscheidern 2a und 2b in die Vakuumanlage. Die Lactidkonzentration im Wasser beträgt 1,5 Gew.-%.

**Beispiel 2:**

Dieses Beispiel illustriert die Lactidabscheidung oberhalb des Drucks am Tripelpunkt. Es entspricht Beispiel 1 mit folgenden Unterschieden:

> Auf der Außenseite der von innen mit Wasser auf 35°C gekühlten Rohre und Platten schlägt sich Dilactid in fester Form nieder. Der Vakuumpumpensatz wird so betrieben, dass am Gasauslass des Lactidabscheiders 2a ein Druck von 4 mbar abs. anliegt. Am Gasauslass des Entmonomerisierers 1 beträgt der Druck infolge von Verlusten im Lactidabscheider 5 mbar.

Das in den Lactidabscheidern anfallende Lactid hat nach Aufschmelzen und Ablassen aus dem Sammelbehälter 9 eine Carboxylgruppen-Konzentration von 50 mmol/kg und bildet eine klare, leicht gelbliche Schmelze. Es wird ohne weitere Reinigung dem frischen Lactid zugesetzt, das als Rohstoff für die Ringöffnungspolymerisation in der Anlage dient. Die Rückführung verändert nicht die Farbe des amorphen PLA-Granulats und die mittlere Molmasse, gemessen über die intrinsische Viskosität gegenüber dem Betrieb der Anlage ohne diese Rückführung.

Die nach der Ringöffnungspolymerisation in die Entmonomerisierung eintretende PLA-Schmelze hat eine Lactid-Konzentration von 4,5 %. Diese Konzentration hat in dem aus der Entmonomerisierung 1 kommenden PLA C1 (Fig. 1) auf eine Lactidkonzentration von 0,32 % abgenommen, gemessen mit Gaschromatographie, nach Abschrecken der Schmelze in Wasser und Granulieren. Die Lactidkonzentration im Wasser aus Behälter 6, gemessen wie in Beispiel 1, beträgt 0,5 Gew.-%.

**Analytische Methoden:**

<u>*1. Carboxylgruppen im Lactid:*</u>

Die Lactidprobe wird in Methanol gelöst. Anschließend wird die Lösung mit 0,1 N benzylalkoholischer KOH-Lösung titriert. Der Endpunkt wird potentiometrisch erfasst.

<u>*2. Restlactidgehalt im PLA:*</u>

Die PLA-Probe wird in Chloroform gelöst und mit Isopropanol gefällt. Das gefällte PLA wird abfiltriert, dabei verbleiben die niedermolekularen Bestandteile in der Lösung. Nach Zugabe von Pentamethylbenzol als internem Standard wird die Lösung im Gaschromatographen auf einer Kapillarsäule DB-5; 15/0,32 in ihre Bestandteile getrennt und detektiert.

*3. Bestimmung der Intrinsischen Lösungsviskosität:*

Die abgewogene Polymermenge wird in einem definierten Volumen Chloroform gelöst. In einem Ubbelohde-Kapillarviskosimeter, das sich in einem auf 20°C +/- 0,1°C eingestellten thermostatisierten Wasserbad befindet, wird die Durchlaufzeit der Lösung und des reinen Lösungsmittels gemessen. Der Quotient aus beiden ist die relative Lösungsviskosität. Sie wird mit der Einpunktmethode nach J. Dorgan et al., J. Polym. Sci.: Part B: Polym. Physics, Vol. 43,3100-3111 (2005), in die intrinsische Viskosität (I.V.) umgerechnet. Die I.V. steht mit dem Gewichtsmittel der Molmasse des Polymers in Verbindung, die mit der sog. Mark-Houwink-Gleichung beschrieben wird. Für das Stoffpaar PLA/Chloroform lautet die Gleichung (J. Dorgan, a.a.O.):

$$I.V. = K * M_w^a, \quad \text{mit } K = 1{,}53 * 10^{-4}, \ a = 0{,}759$$

Legende zu Figur 2

| | |
|---|---|
| TP | Tripelpunkt |
| a | Dampfdruckkurve |
| b | Sublimationsdruckkurve |
| c | Schmelzdruckkurve |
| W→X | Abscheiden unterhalb des Drucks am Tripelpunkt (Desublimieren) |
| W'→ X' | Abscheiden oberhalb des Drucks am Tripelpunkt |
| X/X' → Y | Druckerhöhung nach Ende der Abscheidephase |
| Y → Z | Abschmelzen des Diesters |
| X' → Z' | Abschmelzen des Diesters ohne Druckerhöhung |
| Z/Z' → X/ X' | Evakuieren und Abkühlen nach Abschmelzen und Entleeren des Abscheiders |

## Patentansprüche

**1.** Vorrichtung zur Abtrennung und Rückgewinnung eines cyclischen Diesters der allgemeinen Formel I

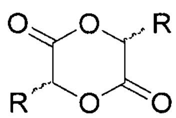

Formel I

wobei R ausgewählt ist aus Wasserstoff oder linearen oder verzweigten aliphatischen Resten mit 1 bis 6 Kohlenstoffatomen, aus Polymerschmelzen, enthaltend den Diester der allgemeinen Formel I, umfassend

a) mindestens eine Entmonomerisierungsvorrichtung (1) zur Entfernung des Diesters der allgemeinen Formel I in gasförmigem Aggregatzustand aus der Polymerschmelze,
b) mindestens eine, der mindestens einen Entmonomerisierungsvorrichtung (1) nachgeschaltete und mit der mindestens einen Entmonomerisierungsvorrichtung (1) in fluidischer Verbindung stehende Abscheidevorrichtung (2a, 2b) zur Abscheidung des Diesters der allgemeinen Formel I, in der der Diester der allgemeinen Formel I über den festen in den flüssigen Aggregatzustand überführt wird, wobei die mindestens eine Abscheidevorrichtung (2a, 2b) derart ausgelegt ist, dass eine Abscheidung des Diesters der allgemeinen Formel I durch Abkühlen auf Temperaturen unterhalb der Tripelpunkttemperatur

i) bei Drücken oberhalb des Tripelpunktdrucks des Diesters der Formel I an einer auf unterhalb der Tripelpunkttemperatur temperierten Oberfläche der mindestens einen Abscheidevorrichtung (2a, 2b) erfolgt, wobei der Diester der Formel I in den festen Aggregatzustand überführt wird, wobei der Druck in der mindestens einen Entmonomerisierungsvorrichtung (1) auf 1,4 bis 100 mbar sowie der Druck in der mindestens einen Abscheidevorrichtung während der Überführung in den festen Aggregatzustand auf 1,4 bis 100 mbar eingestellt wird, oder

ii) bei Drücken unterhalb des Tripelpunktdrucks des Diesters der Formel I an einer auf unterhalb der Tripelpunkttemperatur temperierten Oberfläche der mindestens einen Abscheidevorrichtung (2a, 2b)erfolgt, wobei der Diester der Formel I desublimiert und somit in den festen Aggregatzustand überführt wird, wobei der Druck in der mindestens einen Entmonomerisierungsvorrichtung (1) auf höchstens 1,4 mbar, bevorzugt von 0,01 bis 1,4 mbar, besonders bevorzugt von 0,1 bis 1,4 mbar sowie der Druck in der mindestens einen Abscheidevorrichtung während der Desublimation auf höchstens 1,4 mbar, bevorzugt von 0,01 bis 1,4 mbar, besonders bevorzugt von 0,1 bis 1,4 mbar eingestellt wird,

sowie

c) mindestens eine, der mindestens einen Abscheidevorrichtung (2a, 2b) nachgeschaltete und mit der mindestens einen Abscheidevorrichtung (2a, 2b) in fluidischer Verbindung stehende Vorrichtung (3a, 3b, 3c) zur Erzeugung eines Vakuums,

wobei die mindestens eine Abscheidevorrichtung (2a, 2b) über mindestens einen bodenseitigen Auslass verfügt, dem mindestens ein Sammeltank (9) für den Diester der allgemeinen Formel I nachgeschaltet ist und

wobei der mindestens einen Vorrichtung (3a, 3b, 3c) zur Erzeugung eines Vakuums mindestens eine Vorrichtung (5) zur Erzeugung eines Vorvakuums vorgeschaltet ist, die mit der mindestens einen Vorrichtung (3a, 3b, 3c) zur Erzeugung eines Vakuums in fluidischer Verbindung steht.

2. Vorrichtung nach Anspruch 1, **dadurch gekennzeichnet, dass** mindestens zwei Abscheidevorrichtungen (2a, 2b) umfasst sind, die alternierend betrieben werden können und über ein Dreiwegeventil (10) mit der Entmonomerisierungsvorrichtung (1) und über ein Dreiwegeventil (13) mit mindestens einen Vorrichtung (3a, 3b, 3c) zur Erzeugung eines Vakuums in fluidischer Verbindung stehen.

3. Vorrichtung nach einem der vorhergehenden Ansprüche, **dadurch gekennzeichnet, dass** die mindestens eine Vorrichtung (3a, 3b, 3c) zur Erzeugung eines Vakuums eine Strahlvakuumpumpe, insbesondere eine Dampfstrahlvakuumpumpe oder eine Kaskade aus mindestens zwei, bevorzugt mindestens drei Strahlvakuumpumpen, insbesondere Dampfstrahlvakuumpumpen ist, wobei die Kaskade bevorzugt aus mindestens zwei Strahlvakuumpumpen ohne Zwischenkondensation ausgeführt ist.

4. Vorrichtung nach einem der vorhergehenden Ansprüche, **dadurch gekennzeichnet, dass** der mindestens einen Vorrichtung (3a, 3b, 3c) zur Erzeugung eines Vakuums mindestens ein Kondensator (4), insbesondere ein Oberflächenkondensator nachgeschaltet ist, der mit einem Auslass der mindestens einen Vorrichtung (3a, 3b, 3c) zur Erzeugung eines Vakuums in fluidischer Verbindung steht.

5. Vorrichtung nach vorhergehendem Anspruch, **dadurch gekennzeichnet, dass** dem mindestens einen Kondensator (4) mindestens ein Kondensatsammelbehälter (6) nachgeschaltet ist, der mit dem mindestens einen Kondensator (4) in fluidischer Verbindung steht.

6. Vorrichtung nach einem der vorhergehenden Ansprüche, **dadurch gekennzeichnet, dass** dem mindestens einen Kondensator (4) mindestens eine Vorrichtung (5) zur Erzeugung eines Vorvakuums, insbesondere mindestens eine Wasserringpumpe vorgeschaltet ist, die mit dem mindestens einen Kondensator (4) in fluidischer Verbindung steht.

7. Vorrichtung nach einem der vorhergehenden Ansprüche, **dadurch gekennzeichnet, dass** dem mindestens einen Kondensatsammelbehälter (6), der mindestens einen Vorrichtung (3a, 3b, 3c) zur Erzeugung eines Vakuums und/oder dem mindestens einen Kondensator (4) mindestens eine Reinigungskolonne (7), insbesondere eine Strippkolonne nachgeschaltet ist, die mit dem mindestens einen Kondensatsammelbehälter (6), dem mindestens einen Kondensator (4) und/oder einem Auslass der mindestens einen Vorrichtung (3a, 3b, 3c) zur Erzeugung eines Vakuums in fluidischer Verbindung steht und einen kopfseitigen Einlass (18) für Kondensat und/oder Dämpfe, einen bodenseitigen Gaseinlass (19), einen kopfseitigen Gasauslass (20) sowie einen bodenseitigen Flüssigkeitsauslass (21) aufweist.

**8.** Vorrichtung nach einem der vorhergehenden Ansprüche, **dadurch gekennzeichnet, dass** der mindestens einen Reinigungskolonne (7), insbesondere der Strippkolonne, der mindestens einen Vorrichtung (3a, 3b, 3c) zur Erzeugung eines Vakuums, dem mindestens einen Kondensator (4) und/oder dem mindestens einen Kondensatsammelbehälter (6) mindestens ein Dampferzeuger (8) nachgeschaltet ist, der einen Flüssigkeitszulauf aufweist, der mit der mindestens einen Reinigungskolonne (7), insbesondere der Strippkolonne über den Flüssigkeitsauslass (21), einem Auslass der mindestens einen Vorrichtung (3a, 3b, 3c) zur Erzeugung eines Vakuums, dem mindestens einen Kondensator (4) und/oder dem mindestens einen Kondensatsammelbehälter (6) in fluidischer Verbindung steht.

**9.** Vorrichtung nach einem der beiden vorhergehenden Ansprüche, **dadurch gekennzeichnet, dass** der mindestens eine Dampferzeuger (8)

a) mindestens einen Brenner (22) umfasst, der mindestens eine Zuführung für Brennstoff (G) sowie eine Zuführung (23) von gasförmigem Oxidationsmittel für den Brennstoff, die mit dem kopfseitigen Gasauslass (20) der Reinigungskolonne (7), insbesondere der Strippkolonne in fluidischer Verbindung steht, aufweist; und/oder
b) eine Abführung für erzeugten Dampf (D) aufweist, die mit einer Dampfstrahlvakuumpumpe oder einer Kaskade aus mindestens zwei, bevorzugt mindestens drei Dampfstrahlvakuumpumpen verbunden ist, so dass die Dampfstrahlvakuumpumpe oder die Kaskade aus mindestens zwei, bevorzugt mindestens drei Dampfstrahlvakuumpumpen durch mit vom Dampferzeuger (8) erzeugten Dampf (D) betrieben werden kann.

**10.** Verfahren zur Abtrennung und Rückgewinnung eines cyclischen Diesters der allgemeinen Formel I

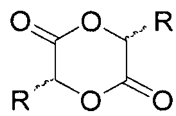

Formel I

wobei R ausgewählt ist aus Wasserstoff oder linearen oder verzweigten aliphatischen Resten mit 1 bis 6 Kohlenstoffatomen, aus Polymerschmelzen, enthaltend den Diester der allgemeinen Formel I, mit einer Vorrichtung nach einem der vorhergehenden Ansprüche, bei dem die Polymerschmelze in mindestens einer Entmonomerisierungsvorrichtung (1) mit Vakuum beaufschlagt und der Diester der allgemeinen Formel I durch Übergang in den gasförmigen Aggregatzustand aus der Polymerschmelze zumindest teilweise oder vollständig abgetrennt und der entfernte gasförmige Diester der allgemeinen Formel I in mindestens einer Abscheidevorrichtung (2a, 2b) durch Abkühlen auf Temperaturen unterhalb der Tripelpunkttemperatur

a) bei Drücken oberhalb des Tripelpunktdrucks des Diesters der Formel I an einer auf unterhalb der Tripelpunkttemperatur temperierten Oberfläche der mindestens einen Abscheidevorrichtung (2a, 2b) in den festen Aggregatzustand überführt wird, wobei t der Druck in der mindestens einen Entmonomerisierungsvorrichtung (1) auf 1,4 bis 100 mbar sowie der Druck in der mindestens einen Abscheidevorrichtung während der Überführung in den festen Aggregatzustand auf 1,4 bis 100 mbar eingestellt wird, oder
b) bei Drücken unterhalb des Tripelpunktdrucks des Diesters der Formel I an einer auf unterhalb der Tripelpunkttemperatur temperierten Oberfläche der mindestens einen Abscheidevorrichtung (2a, 2b) desublimiert und somit in den festen Aggregatzustand überführt wird, wobei der Druck in der mindestens einen Entmonomerisierungsvorrichtung (1) auf höchstens 1,4 mbar, bevorzugt von 0,01 bis 1,4 mbar, besonders bevorzugt von 0,1 bis 1,4 mbar sowie der Druck in der mindestens einen Abscheidevorrichtung während der Desublimation auf höchstens 1,4 mbar, bevorzugt von 0,01 bis 1,4 mbar, besonders bevorzugt von 0,1 bis 1,4 mbar eingestellt wird,

**dadurch gekennzeichnet, dass** anschließend eine Verflüssigung und ein Austrag des Diesters der allgemeinen Formel I aus der mindestens einen Abscheidevorrichtung (2a, 2b) erfolgt, wobei Diester der allgemeinen Formel I aus der mindestens einen Abscheidevorrichtung (2a, 2b) über mindestens einen bodenseitigen Auslass ausgetragen wird und in einem nachgeschalteten Sammeltank (9) gesammelt wird,
wobei dem Vakuum,
das durch mindestens eine Vorrichtung (3a, 3b, 3c) zur Erzeugung eines Vakuums erzeugt wird,
ein Vorvakuum vorgeschaltet ist,
das durch mindestens eine Vorrichtung (5) zur Erzeugung eines Vorvakuums, die mit der mindestens einen Vorrichtung (3a, 3b, 3c) zur Erzeugung eines Vakuums in fluidischer Verbindung steht, erzeugt wird.

**11.** Verfahren nach vorhergehendem Anspruch, **dadurch gekennzeichnet, dass** die Mittel der mindestens einen Abscheidevorrichtung (2a, 2b) zur Desublimation oder Überführung in den festen Aggregatzustand des Diesters der allgemeinen Formel I alternierend mit einem auf unterhalb der Tripelpunkttemperatur temperierten Medium (A) und zur Überführung in den flüssigen Aggregatzustand mit einem auf oberhalb der Tripelpunkttemperatur temperierten Medium (B) durchströmt werden, wobei bevorzugt innerhalb der mindestens einen Abscheidevorrichtung (2a, 2b) während der Desublimation oder Überführung in den festen Aggregatzustand des Diesters der allgemeinen Formel I ein im Vergleich zur Überführung in den flüssigen Aggregatzustand verminderter Druck eingestellt wird, wobei bevorzugt der Druck bei der Überführung in den flüssigen Aggregatzustand auf mindestens 2,5 mbar, weiter bevorzugt von 2,5 bis 1050 mbar, besonders bevorzugt von 10 bis 50 mbar eingestellt wird.

**12.** Verfahren nach einem der beiden vorhergehenden Ansprüche, **dadurch gekennzeichnet, dass** mindestens zwei Abscheidevorrichtungen (2a, 2b) umfasst sind, die alternierend betrieben werden.

**13.** Verfahren nach einem der Ansprüche 10 bis 12, **dadurch gekennzeichnet, dass** die aus der mindestens einen Vorrichtung (3a, 3b, 3c) zur Erzeugung eines Vakuums, bevorzugt der Strahlvakuumpumpe, insbesondere der Dampfstrahlvakuumpumpe oder einer Kaskade aus mindestens zwei, bevorzugt mindestens drei Strahlvakuumpumpen, insbesondere Dampfstrahlvakuumpumpen austretenden Dämpfe in dem mindestens einen Kondensator (4) kondensiert werden und bevorzugt in den mindestens einen Kondensatsammelbehälter (6) überführt werden.

**14.** Verfahren nach vorhergehendem Anspruch, **dadurch gekennzeichnet, dass** das Kondensat kopfseitig in die mindestens eine Reinigungskolonne (7), bevorzugt die mindestens eine Strippkolonne aufgegeben wird und im Gegenstrom mit einem Reinigungsgas, bevorzugt Luft beaufschlagt wird, wobei bevorzugt

a) das aus der mindestens einen Reinigungskolonne (7), bevorzugt der mindestens einen Strippkolonne bodenseitig (21) entnommene Kondensat dem mindestens einen Verdampfer (8) zugeführt und dort verdampft wird, wobei bevorzugt der entstehende Dampf zum Betreiben der Dampfstrahlvakuumpumpe oder der Kaskade aus mehreren Dampfstrahlvakuumpumpen verwendet wird, und/oder
b) das aus dem kopfseitigen Gasauslass (20) entnommenen Reinigungsgas zusammen mit dem Oxidationsmittel für den Brennstoff (G) in den mindestens einen Brenner (22) des mindestens einen Dampferzeugers (8) aufgegeben wird.

**15.** Verfahren nach einem der Ansprüche 10 bis 14, **dadurch gekennzeichnet, dass** die Polymerschmelze eine Schmelze eines Polyesters, insbesondere Polylactid, Polyglycolid und/oder Copolyester hiervon und der Diester der allgemeinen Formel I Lactid und/oder Glycolid ist.

## Claims

**1.** Device for separating and recovering a cyclic diester of general Formula I

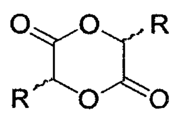

Formula 1

R being selected from hydrogen or linear or branched aliphatic radicals with 1 to 6 carbon atoms, from polymer melts, comprising the diester of general formula I, which comprises

a) at least one demonomerisation device (1) for removing the diester of general Formula I in gaseous aggregate state from the polymer melt,
b) at least one separation device (2a, 2b), which is connected subsequent to the at least one demonomerisation device (1) and which is in fluidic connection with the at least one demonomerisation device (1), for separating the diester of general Formula I, in which the diester of general Formula I is converted via the solid into the

liquid aggregate state, wherein the at least one separation device (2a, 2b) is designed in a way that the separation of the diester of general Formula I is effected

i) at pressures above the triple point pressure of the diester of Formula I on a surface, temperature-controlled to below the triple point temperature, of the at least one separation device (2a, 2b), wherein the diester of Formula I is converted into the solid aggregate state wherein the pressure in the at least one demonomerisation device (1) being set at 1.4 to 100 mbar and also the pressure in the at least one separation device during the conversion into the solid aggregate state being set at 1.4 to 100 mbar,
ii) at pressures below the triple point pressure of the diester of Formula I on a surface, temperature-controlled to below the triple point temperature, of the at least one separation device (2a, 2b), wherein the diester of Formula I is desublimated and hence is converted in the solid aggregate state, wherein the pressure in the at least one demonomerisation device (1) being set at 1.4 mbar, wherein the pressure in the at least one demonomerisation device (1) being set at at most 1.4 mbar, preferably from 0.01 to 1.4 mbar, particularly preferred from 0.1 to 1.4 mbar and also the pressure in the at least one separation device during the desublimation being set at at most 1.4 mbar, preferably from 0.01 to 1.4 mbar, particularly preferred from 0.1 to 1.4 mbar, and also

c) at least one device (3a, 3b, 3c) for producing a vacuum which is connected subsequent to the at least one separation device (2a, 2b) and which is in fluidic connection with the at least one separation device (2a, 2b), wherein the at least one separation device (2a, 2b) has at least one base-side outlet, subsequent to which at least one collection tank (9) for the diester of general Formula I is connected and
wherein in front of the at least one device (3a, 3b, 3c) for producing a vacuum at least one device (5) for producing an initial vacuum is connected, which is in fluidic connection with the at least one device (3a, 3b, 3c) for producing a vacuum.

2. Device according to claim 1, **characterised in that** at least two separation devices (2a, 2b) are comprised, which can be operated alternately and are in fluidic connection via a three-way valve (10) with the demonomerisation device (1) and via a three-way valve (13) with at least one device (3a, 3b, 3c) for producing a vacuum.

3. Device according to one of the preceding claims, **characterised in that** the at least one device (3a, 3b, 3c) for producing a vacuum is an ejector pump, in particular a steam ejector pump, or a cascade of at least two, preferably at least three, ejector pumps, in particular steam ejector pumps, the cascade preferably of at least two ejector pumps being designed without intermediate condensation.

4. Device according to one of the preceding claims, **characterised in that**, subsequent to the at least one device (3a, 3b, 3c) for producing a vacuum, at least one condenser (4), in particular a surface condenser, is connected, which is in fluidic connection with an outlet of the at least one device (3a, 3b, 3c) for producing a vacuum.

5. Device according to the preceding claim, **characterised in that**, subsequent to the at least one condenser (4), at least one condensate-collecting container (6) is connected, which is in fluidic connection with the at least one condenser (4).

6. Device according to one of the preceding claims, **characterised in that**, in front of the at least one condenser (4) at least one device (5) for producing an initial vacuum, in particular at least one water-ring pump, is connected, which is in fluidic connection with the at least one condenser (4).

7. Device according to one of the preceding claims, **characterised in that**, subsequent to the at least one condensate-collecting container (6), to the at least one device (3a, 3b, 3c) for producing a vacuum and/or to the at least one condenser (4) at least one purification column (7), in particular a stripping column, is connected, which is in fluidic connection with the at least one condensate-collecting container (6), with the at least one condenser (4) and/or with an outlet of the at least one device (3a, 3b, 3c) for producing a vacuum, and has a top-side inlet (18) for condensate and/or vapours, a base-side gas inlet (19), a top-side gas outlet (20) and also a base-side liquid outlet (21).

8. Device according to one of the preceding claims, **characterised in that**, subsequent to the at least one purification column (7), in particular to the stripping column, to the at least one device (3a, 3b, 3c) for producing a vacuum, to the at least one condenser (4) and/or to the at least one condensate-collecting container (6), at least one steam producer (8) is connected, which has a liquid inflow which is in fluidic connection with the at least one purification column (7), in particular the stripping column, via the liquid outlet (21), with an outlet of the at least one device (3a,

3b, 3c) for producing a vacuum, with the at least one condenser (4) and/or with the at least one condensate-collecting container (6).

**9.** Device according to one of the two preceding claims, **characterised in that** the at least one steam producer (8)

a) comprises at least one burner (22) which has at least one supply line for fuel (G) and also a supply line (23) of gaseous oxidant for the fuel, which is in fluidic connection with the top-side gas outlet (20) of the purification column (7), in particular of the stripping column; and/or
b) has a discharge for produced steam (D), which is connected to a steam ejector pump or to a cascade of at least two, preferably at least three, steam ejector pumps so that the steam ejector pump or the cascade of at least two, preferably at least three, steam ejector pumps can be operated by steam (D) produced by the steam producer (8).

**10.** Method for separating and recovering a cyclic diester of general Formula I,

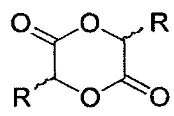

Formula I

R being selected from hydrogen or linear or branched aliphatic radicals with 1 to 6 carbon atoms, from polymer melts comprising the diester of general Formula I, with a device according to one of the preceding claims in which the polymer melt is supplied with vacuum in at least one demonomerisation device (1) and the diester of general Formula I is separated at least partially or completely from the polymer melt by transition into the gaseous aggregate state and the removed gaseous diester of general Formula I, in at least one separation device (2a, 2b) by cooling to temperatures below the triple point temperature,

a) is converted into the solid aggregate state at pressures above the triple point pressure of the diester of Formula I on a surface, temperature-controlled to below the triple point temperature, of the at least one separation device (2a, 2b), preferably the pressure in the at least one demonomerisation device (1) being set at 1.4 to 100 mbar and also the pressure in the at least one separation device during the conversion into the solid aggregate state being set at 1.4 to 100 mbar, or
b) is desublimated at pressures below the triple point pressure of the diester of Formula I on a surface, temperature-controlled to below the triple point temperature, of the at least one separation device (2a, 2b) and hence converted into the solid aggregate state, preferably the pressure in the at least one demonomerisation device (1) being set at at most 1.4 mbar, preferably from 0.01 to 1.4 mbar, particularly preferred from 0.1 to 1.4 mbar and also the pressure in the at least one separation device during the desublimation being set at at most 1.4 mbar, preferably from 0.01 to 1.4 mbar, particularly preferred from 0.1 to 1.4 mbar,

**characterised in that** subsequently liquefaction and discharge of the diester of general Formula 1 from the at least separation device (2a, 2b) is effected, wherein the diester of general Formual I is discharged from the at least one separation device (2a, 2b) via at least one base-side outlet and collected in at least one collection tank (9), wherein
in front of the vaccum, which is produced by the at least one device (3a, 3b, 3c) for producing a vacuum,
an initial vacuum is connected
which is produced by at least one device (5) for producing an initial vacuum, which is in fluidic connection with the at least one device (3a, 3b, 3c) for producing a vacuum.

**11.** Method according to the preceding claim, **characterised in that** the means of the at least one separation device (2a, 2b) for desublimation or conversion into the solid aggregate state of the diester of general Formula I are flowed through alternately with a medium (A) temperature-controlled to below the triple point temperature and for conversion into the liquid aggregate state with a medium (B) temperature-controlled to above the triple point temperature, wherein preferably within the at least one separation device (2a, 2b) during desublimation or conversion into the solid aggregate state of the diester of general Formula I, a pressure which is reduced in comparison to the conversion into the liquid aggregate state is set, the pressure during conversion into the liquid aggregate state being set preferably

to at least 2.5 mbar, further preferred from 2.5 to 1,050 mbar, particularly preferred from 10 to 50 mbar.

**12.** Method according to one of the two preceding claims, **characterised in that** at least two separation devices (2a, 2b) are included, which are operated alternately.

**13.** Method according to one of the claims 11 to 13, **characterised in that** the steam emerging from the at least one device (3a, 3b, 3c) for producing a vacuum, preferably from the ejector pump, in particular from the steam ejector pump, or from a cascade of at least two, preferably at least three, ejector pumps, in particular from steam ejector pumps, is condensed in the at least one condenser (4) and preferably transferred into the at least one condensate-collecting container (6).

**14.** Method according to the preceding claim, **characterised in that** the condensate is fed, at the top-side, into the at least one purification column (7), preferably into the at least one stripping column and supplied in counterflow with a purification gas, preferably air, wherein preferably

a) the condensate which is removed from the at least one purification column (7), preferably from the at least one stripping column at the base-side (21), is supplied to the at least one evaporator (8) and evaporated there, the resulting steam preferably being used for operating the steam ejector pump or the cascade of a plurality of steam ejector pumps, and/or
b) the purification gas which is removed from the top-side gas outlet (20) is fed, together with the oxidant for the fuel (G), into the at least one burner (22) of the at least one steam producer (8).

**15.** Method according to one of the claims 10 to 14, **characterised in that** the polymer melt is a melt of a polyester, in particular polylactide, polyglycolide and/or copolyesters hereof, and the diester of general Formula I is lactide and/or glycolide.

## Revendications

**1.** Dispositif pour la séparation et la récupération d'un diester cyclique de formule générale I

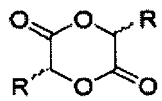

Formule I

dans laquelle R est choisi parmi l'atome d'hydrogène ou les radicaux aliphatiques linéaires ou ramifiés ayant 1 à 6 atomes de carbone, à partir de masses fondues de polymères contenant le diester de formule générale I, comprenant

a) au moins un dispositif de démonomérisation (1) pour éliminer de la masse fondue de polymères le diester de formule générale I à un état d'agrégat gazeux,
b) au moins un dispositif de précipitation (2a, 2b), disposé en aval de l'au moins un dispositif de démonomérisation (1) et en liaison fluidique avec l'au moins un dispositif de démonomérisation (1), pour provoquer la précipitation du diester de formule générale I, dans lequel ledit ester de formule générale I est, en passant par un état d'agrégat solide, converti à un état d'agrégat liquide, l'au moins un dispositif de précipitation (2a, 2b) étant conçu de telle sorte qu'il en résulte une précipitation du diester de formule générale I par refroidissement à des températures inférieures à la température du point triple

i) pour des pressions supérieures à la pression du point triple du diester de formule I, sur une surface, portée à l'équilibre de température en-dessous de la température du point triple, de l'au moins un dispositif de précipitation (2a, 2b), le diester de formule I étant converti à un état d'agrégat solide, la pression dans l'au moins un dispositif de démonomérisation (1) étant ajustée à 1,4 à 100 mbar, ainsi que la pression dans l'au moins un dispositif de précipitation étant, pendant la conversion, ajusté, à l'tat d'agrégat solide, à 1,4 à 100 mbar, ou
ii) pour des pressions inférieures à la pression du point triple du diester de formule I, sur une surface, portée à l'équilibre de température en-dessous de la température du point triple, de l'au moins un dispositif de précipitation (2a, 2b), le diester de formule I étant désublimé et ainsi converti à un état d'agrégat solide, la

pression dans l'au moins un dispositif de démonomérisation (1) étant ajustée à un maximum de 1,4 mbar, de préférence de 0,01 à 1,4 mbar, d'une manière particulièrement préférée de 0,1 à 1,4 mbar, et la pression dans l'au moins un dispositif de précipitation étant, pendant la désublimation, ajustée à un maximum de 1,4 mbar, de préférence de 0,01 à 1,4 mbar, d'une manière particulièrement préférée de 0,1 à 1,4 mbar,

ainsi que
c) au moins un dispositif (3a, 3b, 3c), pour la production d'un vide, disposé en aval de l'au moins un dispositif de précipitation (2a, 2b) et en liaison fluidique avec l'au moins un dispositif de précipitation (2a, 2b),

dans lequel l'au moins un dispositif de précipitation (2a, 2b) dispose d'au moins un orifice de sortie au fond, en aval duquel est disposé au moins un réservoir d'accumulation (9) pour le diester de formule générale I, et
dans lequel en amont de l'au moins un dispositif (3a, 3b, 3c) pour la production d'un vide est disposé au moins un dispositif (5) pour la production d'un prévide, qui est en liaison fluidique avec l'au moins un dispositif (3a, 3b, 3c) pour la production d'un vide.

2. Dispositif selon la revendication 1, **caractérisé en ce qu'**il comprend au moins deux dispositifs de précipitation (2a, 2b), qui peuvent être exploités en alternance et sont en liaison fluidique par l'intermédiaire d'une vanne à trois voies (10) avec le dispositif de démonomérisation (1), et par l'intermédiaire d'une vanne à trois voies (13) avec au moins un dispositif (3a, 3b, 3c) pour la production d'un vide.

3. Dispositif selon l'une des revendications précédentes, **caractérisé en ce que** l'au moins un dispositif (3a, 3b, 3c) pour la production d'un vide est une pompe à vide à jet, en particulier une pompe à vide à jet de vapeur ou une cascade d'au moins deux, de préférence d'au moins trois pompes à vide à jet, en particulier pompes à vide à jet de vapeur, la cascade étant de préférence conçue à partir d'au moins deux pompes à vide à jet sans condensation intermédiaire.

4. Dispositif selon l'une des revendications précédentes, **caractérisé en ce qu'**en aval de l'au moins un dispositif (3a, 3b, 3c) pour la production d'un vide est disposé au moins un condenseur (4), en particulier un condenseur à surface, qui est en relation fluidique avec un orifice de sortie de l'au moins un dispositif (3a, 3b, 3c) pour la production d'un vide.

5. Dispositif selon la revendication précédente, **caractérisé en ce qu'**en aval de l'au moins un condenseur (4) est disposé au moins un récipient collecteur de purge (6), qui est en relation fluidique avec l'au moins un condenseur (4).

6. Dispositif selon l'une des revendications précédentes, **caractérisé en ce qu'**en amont de l'au moins un condenseur (4) est disposé au moins un dispositif (5) pour la production d'un prévide, en particulier au moins une pompe à anneau d'eau, qui est en liaison fluidique avec l'au moins un condenseur (4).

7. Dispositif selon l'une des revendications précédentes, **caractérisé en ce qu'**en aval de l'au moins un récipient collecteur de purge (6), de l'au moins un dispositif (3a, 3b, 3c) pour la production d'un vide et/ou de l'au moins un condenseur (4) est disposée au moins une colonne de purification (7), en particulier une colonne de fractionnement, qui est en liaison fluidique avec l'au moins un récipient collecteur de purge (6), avec l'au moins un condenseur (4) et/ou avec un orifice de sortie de l'au moins un dispositif (3a, 3b, 3c) pour la production d'un vide, la colonne de rectification présentant un orifice d'entrée (18) en tête pour le condensat et/ou les vapeurs, un orifice d'entrée des gaz (19) au fond, un orifice de sortie des gaz (20) en tête, ainsi qu'un orifice de sortie de liquide (21) au fond.

8. Dispositif selon l'une des revendications précédentes, **caractérisé en ce qu'**en aval de l'au moins une colonne de purification (7), en particulier de la colonne de rectification, de l'au moins un dispositif (3a, 3b, 3c) pour la production d'un vide, de l'au moins un condenseur (4) et/ou de l'au moins un récipient collecteur de purge (6) est disposé au moins un générateur de vapeur (8), qui présente une amenée de liquide, qui est en liaison fluidique avec l'au moins une colonne de purification (7), en particulier l'au moins une colonne de rectification, par l'intermédiaire de l'amenée de liquide (21), avec un orifice de sortie de l'au moins un dispositif (3a, 3b, 3c) pour la production d'un vide, de l'au moins un condenseur (4) et/ou de l'au moins un récipient collecteur de purge (6).

9. Dispositif selon l'une des deux revendications précédentes, **caractérisé en ce que** l'au moins un générateur de vapeur (8)

a) comprend au moins un brûleur (22), qui présente au moins une amenée pour le combustible (G) ainsi qu'une amenée (23) pour un oxydant gazeux pour le combustible, qui est en liaison fluidique avec l'orifice de sortie de

gaz (20) en tête de la colonne de purification (7), en particulier de la colonne de rectification ; et/ou
b) présente une évacuation pour la vapeur produite (D), qui est reliée à une pompe à vide à jet de vapeur ou à une cascade d'au moins deux, de préférence trois pompes à vide à jet de vapeur, de telle sorte que la pompe à vide à jet de vapeur ou la cascade d'au moins deux, de préférence trois pompes à vide à jet de vapeur, puisse être exploitée par la vapeur (D) produite par le générateur de vapeur (8).

**10.** Procédé pour la séparation et la récupération d'un diester cyclique de formule générale I

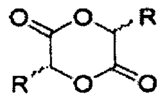

Formule I

dans laquelle R est choisi parmi l'atome d'hydrogène ou les radicaux aliphatiques linéaires ou ramifiés ayant 1 à 6 atomes de carbone, à partir de masses fondues de polymères contenant le diester de formule générale I, à l'aide d'un dispositif selon l'une des revendications précédentes, dans lequel la masse fondue de polymères est, dans au moins un dispositif de démonomérisation (1), soumise à un vide, et le diester de formule générale I est au moins en partie ou en totalité séparé, par transition à un état d'agrégat gazeux à partir de la masse fondue de polymères, et le diester gazeux éliminé de formule générale I est, dans au moins un dispositif de précipitation (2a, 2b), par refroidissement à des températures inférieures à la température du point triple,

a) pour des pressions supérieures à la pression du point triple du diester de formule I, converti à un état d'agrégat solide sur une surface, portée à l'équilibre de température en-dessous de la température du point triple, de l'au moins un dispositif de précipitation (2a, 2b), la pression étant ajustée dans l'au moins un dispositif de démonomérisation (1) à 1,4 à 100 mbar, et, dans l'au moins un dispositif de précipitation, pendant la conversion à un état d'agrégat solide, à 1,4 à 100 mbar, ou
b) pour des pressions inférieures à la pression du point triple du diester de formule I, désublimé sur une surface, portée à l'équilibre de température en-dessous de la température du point triple, de l'au moins un dispositif de précipitation (2a, 2b), et ainsi converti à un état d'agrégat solide, la pression étant ajustée, dans l'au moins un dispositif de démonomérisation (1), au maximum à 1,4 mbar, de préférence à 0,01 à 1,4 mbar, d'une manière particulièrement préférée à 0,1 à 1,4 mbar, ainsi que, dans l'au moins un dispositif de précipitation, pendant la désublimation, au maximum à 1,4 mbar, de préférence à 0,01 à 1,4 mbar, d'une manière particulièrement préférée à 0,1 à 1,4 mbar,

**caractérisé en ce que** l'on procède ensuite à une liquéfaction et à une décharge du diester de formule générale I à partir de l'au moins un dispositif de précipitation (2a, 2b), le diester de formule générale I étant déchargé de l'au moins un dispositif de précipitation (2a, 2b) par au moins un orifice de sortie au fond, et étant accumulé dans un réservoir d'accumulation (9) monté en aval,
un prévide qui est produit par au moins un dispositif (5) pour la production d'un prévide, qui est en liaison fluidique avec au moins un dispositif (3a, 3b, 3c) pour la production d'un vide, étant disposé en amont du vide qui est produit par au moins un dispositif (3a, 3b, 3c) pour la production d'un vide.

**11.** Procédé selon la revendication précédente, **caractérisé en ce que** les moyens de l'au moins un dispositif de précipitation (2a, 2b) sont traversés, en alternance, pour la désublimation ou la conversion à un état d'agrégat solide du diester de formule générale I, par un fluide (A) porté à l'équilibre de température en-dessous de la température du point triple, et, pour la conversion à un état d'agrégat solide, par un fluide (B) porté à l'équilibre de température au-dessus de la température du point triple, dans lequel de préférence, à l'intérieur de l'au moins un dispositif de précipitation (2a, 2b), pendant la désublimation ou la conversion à un état d'agrégat solide du diester de formule générale I, on ajuste une pression diminuée par rapport à la conversion à un état d'agrégat liquide, la pression étant ajustée de préférence, lors de la conversion à un état d'agrégat liquide, à au moins 2,5 mbar, d'une manière plus préférée de 2,5 à 1050 mbar, d'une manière particulièrement préférée de 10 à 50 mbar.

**12.** Procédé selon l'une des revendications précédentes, **caractérisé en ce qu'**il comprend au moins deux dispositifs de précipitation (2a, 2b), qui sont exploités en alternance.

**13.** Procédé selon l'une des revendications 10 à 12, **caractérisé en ce que** les vapeurs déchargées de l'au moins un dispositif (3a, 3b, 3c) pour la production d'un vide, de préférence de la pompe à vide à jet, en particulier de la pompe

à vide à jet de vapeur ou d'une cascade d'au moins deux, de préférence d'au moins trois pompes à vide à jet, en particulier pompes à vide à jet de vapeur, sont condensées dans l'au moins un condenseur (4), et de préférence transférées dans l'au moins un récipient collecteur de purge (6).

**14.** Procédé selon la revendication précédente, **caractérisé en ce que** le condensat est introduit en tête dans l'au moins une colonne de purification (7), de préférence dans l'au moins une colonne de rectification, et reçoit à contre-courant un gaz de purification, de préférence de l'air, dans lequel de préférence

a) le condensat soutiré au fond (21) de l'au moins une colonne de purification (7), de préférence de l'au moins une colonne de rectification, est envoyé à l'au moins un évaporateur (8) et y est évaporé, la vapeur produite étant de préférence utilisée pour exploiter la pompe à vide à jet de vapeur ou la cascade de plusieurs pompes à vide à jet de vapeur, et/ou
b) le gaz de purification soutiré de l'orifice de sortie du gaz (20) en tête est, en même temps que l'oxydant pour le combustible (G), introduit dans au moins un brûleur (22) de l'au moins un générateur de vapeur (8).

**15.** Procédé selon l'une des revendications 10 à 14, **caractérisé en ce que** la masse fondue de polymères est une masse d'un polyester, en particulier d'un polylactide, d'un polyglycolide et/ou d'un copolyester de ceux-ci, et le diester de formule générale I est un lactide et/ou un glycolide.

Fig. 1

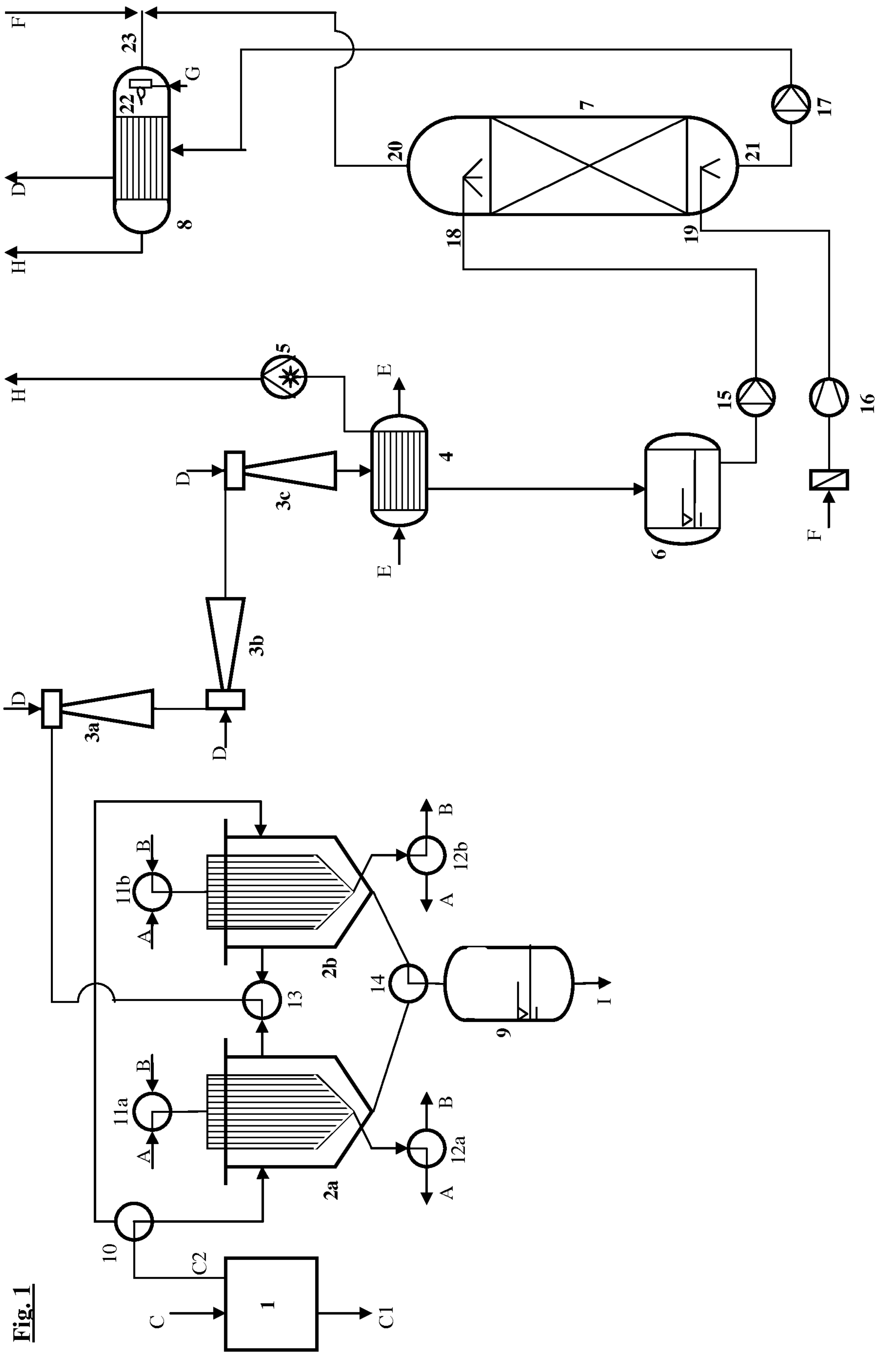
1
C
C1
C2
10
2a
2b
11a
11b
12a
12b
13
14
9
I
A
B
3a
3b
3c
D
4
E
5
H
6
15
16
F
7
17
18
19
20
21
8
22
23
G

FIGUR 2

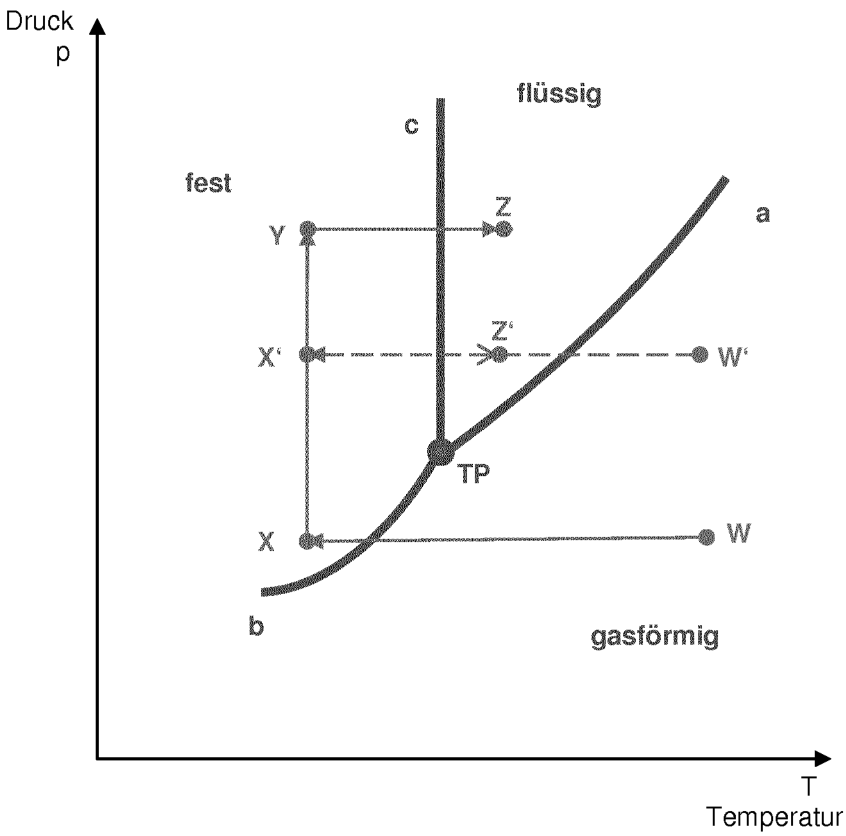

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19630121 A1
- EP 0499747 A2
- WO 9836012 A
- EP 2055730 A2
- US 20090299018 A1
- US 5866677 A
- WO 2012110117 A1
- US 20090299018 A


### In der Beschreibung aufgeführte Nicht-Patentliteratur


- **J. DORGAN et al.** *J. Polym. Sci.: Part B: Polym. Physics,* 2005, vol. 43, 3100-3111